# EUROPEAN PATENT APPLICATION

(11) **EP 4 782 556 A2**
(43) Date of publication of application: **29.07.2026**
(21) Application number: 26166367.8
(22) Date of filing: 22.03.2022
(51) Int. Cl.: C12Q 1/6897

(54) **MULTI-INPUT MIRNA SENSING WITH CONSTITUTIVE ERNS TO REGULATE MULTI-OUTPUT GENE EXPRESSION IN MAMMALIAN CELLS**

(30) Priority: 22.03.2021 US 202163164282 P
(62) Divisional of application: 22776431.3
(71) Applicant: Massachusetts Institute of Technology, Cambridge, MA 02139 (US)
(72) Inventor: WEISS, Ron, Newton, MA 02459 (US); MISHRA, Deepak, Cambridge, MA 02141 (US); PERY, Erez, Nashua, NH 03062 (US); XU, Wenlong, Cambridge, MA 02139 (US); WANG, Lei, Cambridge, MA 02139 (US)
(74) Representative: Hoffmann Eitle

(57) **Abstract**

Provided herein are sequestrons for detecting an miRNA profile indicative of a cell state and expressing an output molecule in cells having such an miRNA profile. Also provided are methods of using sequestrons provided herein.

## Description

### RELATED APPLICATION

This application claims the benefit under 35 U.S.C. § 119(e) of U.S. provisional Application No. 63/164,282 filed March 22, 2021, which is incorporated by reference herein in its entirety.

### BACKGROUND

The ability to classify individual cell types in complex biological samples (e.g., in a tissue or during cellular differentiation) can be achieved using the unique expression patterns of microRNAs (miRNAs) in specific cell types or in individual cells. The miRNA profile of a given cell thus provides a signature of its phenotype and/or stage of development.

### SUMMARY

Provided herein are sequestrons designed to express an output molecule *(e.g.,* a detectable molecule, a transcription factor, or a therapeutic molecule) under desired conditions. Such desired conditions include the presence of one or more miRNAs indicative of a desired cell state, absence of one or more miRNAs indicative of an undesired cell state, and/or a specific miRNA profile indicative of a desired cell state. Sensor circuits of certain sequestrons provided herein encode repressors that are translationally regulated by the presence of a desired miRNA, such that the repressor is not produced in the presence of one or more miRNAs that indicate a desired cell state. Signal circuits of the sequestrons provided herein encode output molecules that are produced only in the absence of a repressor, and optionally the absence of one or more undesired miRNAs. The sequestrons provided herein are regulated translationally, with RNA being transcribed constitutively from both sensor circuits and signal circuits, but translated only in the absence of negative regulation by miRNA and/or repressor activity. Without wishing to be bound by theory, it is believed that translational regulation of both repressor production and output molecule expression allows sequestrons to respond efficiently to changes in the miRNA profile in a cell. Because constitutively transcribed mRNA encoding the output molecule is consistently available in sequestron-expressing cells, output molecule translation can be increased quickly in response to decreased abundance of inhibitory miRNA or repressor. Conversely, constitutively transcribed mRNA encoding the repressor is also consistently available for translation following relaxation of miRNA-mediated downregulation, and so the repressor can be quickly translated to repress output molecule translation in response to decreased abundance of undesired miRNA. Accordingly, the present disclosure provides, in some aspects, a sequestron comprising:
(i) a sensor circuit comprising a first constitutive promoter operably linked to a nucleic acid sequence encoding:
   (a) a nucleic acid sequence encoding a repressor; and
   (b) one or more target sequences for a first set of one or more miRNAs; and
(ii) a signal circuit comprising a second constitutive promoter operably linked to a nucleic acid sequence encoding:
   (a) a repressor recognition sequence that is capable of being bound or cleaved by the repressor of (i)(a); and
   (b) a nucleic acid sequence encoding an output molecule.

In some embodiments, the one or more target sequences for the first set of one or more miRNAs of (i)(b) are downstream from the nucleic acid sequence encoding the repressor of (i)(a).

In some embodiments, the repressor recognition sequence of (ii)(a) is upstream from the nucleic acid sequence encoding the output molecule of (ii)(b).

In some embodiments, the nucleic acid sequence encoded by the signal circuit of (ii) further comprises one or more target sequences for a second set of one or more miRNAs.

In some embodiments, the one or more target sequences for the second set of one or more miRNAs are downstream from the nucleic acid sequence encoding the output molecule.

In some embodiments, the sequestron comprises a plurality of the signal circuit of (ii).

In some embodiments, each of the plurality of signal circuits comprises a target sequence for a different miRNA of the second set of miRNAs, wherein the target sequence is not present on the other signal circuits.

In some embodiments, the sequestron comprises a plurality of the sensor circuit of (i).

In some embodiments, each of the plurality of sensor circuits comprises a target sequence for a different miRNA of the first set of miRNAs, wherein the target sequence is not present on the other sensor circuits.

In some embodiments, the repressor is an endoribonuclease, an RNAi molecule, or a ribozyme.

In some embodiments, the repressor is a CRISPR endoribonuclease, and the repressor recognition sequence is a CRISPR endoribonuclease recognition sequence.

In some embodiments, the CRISPR endoribonuclease is Cas6, Csy4, CasE, Cse3, LwaCas13a, PspCas13b, RanCas13b, PguCas13b, or RfxCas13d.

In some embodiments, the first and/or second constitutive promoter is an hEF1-alpha promoter.

In some aspects, the disclosure provides a composition comprising a plurality of the sequestrons provided herein, where:
(A) the nucleic acid sequence of (i)(a) of each of the plurality of sequestrons encodes a different repressor;
(B) the repressor recognition sequence of (ii)(a) of each of the plurality of sequestrons comprises a different nucleic acid sequence;
(C) the repressor encoded by the sensor circuit of each of the plurality of sequestrons is capable of binding or cleaving the repressor recognition sequence of the signal circuit of the same sequestron; and
(D) the repressor encoded by the sensor circuit of each sequestron is not capable of binding or cleaving the repressor recognition sequence of a different sequestron.

In some aspects, the disclosure provides a composition comprising any of the sequestrons provided herein, or a plurality of the sequestrons provided herein.

In some embodiments, the composition further comprises a pharmaceutically acceptable excipient.

In some aspects, the disclosure provides a cell comprising any of the sequestrons provided herein, or a plurality of any of the sequestrons provided herein.

In some embodiments, the cell is a prokaryotic cell.

In some embodiments, the prokaryotic cell is a bacterial cell.

In some embodiments, the cell is a eukaryotic cell.

In some embodiments, the eukaryotic cell is a plant cell, an insect cell, or a mammalian cell.

In some embodiments, the eukaryotic cell is a human cell.

In some embodiments, the cell is a diseased cell.

In some embodiments, the cell is a cancer cell.

In some embodiments, the cell expresses any one of the first set of microRNAs.

In some aspects, the disclosure provides a method comprising maintaining, in culture, any of the cells provided herein.

In some embodiments, the method further comprises detecting the output molecule.

In some embodiments, the method further comprises classifying the cell.

In some aspects, the disclosure provides a method comprising delivering any of the sequestrons or compositions provided herein to a cell.

In some embodiments, the method further comprises detecting the output molecule.

In some embodiments, the method further comprises classifying the cell.

In some aspects, the disclosure provides a method of treating a disease or disorder, the method comprising delivering any of the sequestrons or compositions provided herein to a subject in need thereof, wherein the output molecule is a therapeutic molecule that treats the disease or disorder.

In some aspects, the disclosure provides a method of diagnosing a disease or disorder, the method comprising administering an effective amount of any of the sequestrons or compositions provided herein to a subject.

In some embodiments, the method further comprises detecting the output molecule.

### BRIEF DESCRIPTION OF THE DRAWINGS

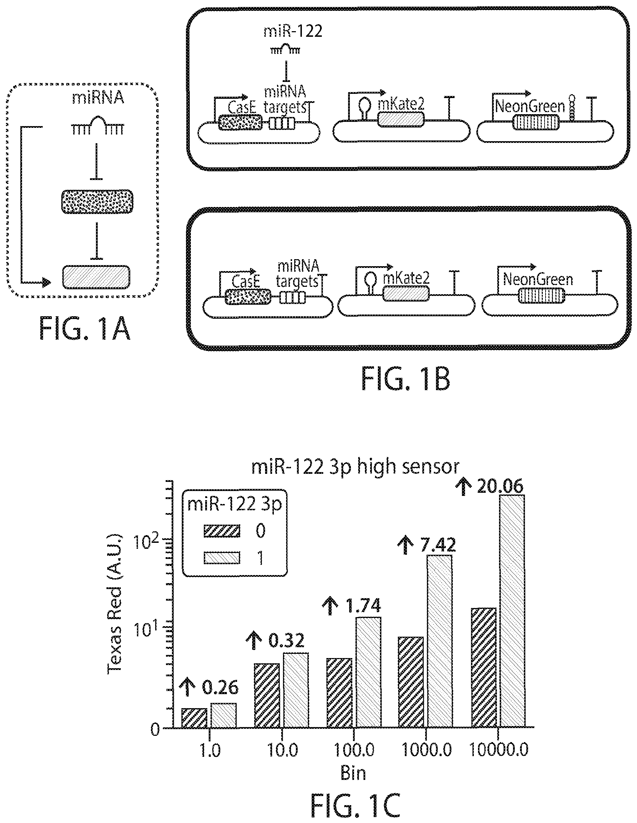

**FIGs. 1A-1C** show the design and validation of sequestrons that produce an output molecule in the presence of an miRNA signal. **FIG. 1A** shows that the presence of an miRNA inhibits a repressor of an output molecule, and thus the presence of the miRNA results in production of the output molecule. **FIG. 1B** shows a detailed design of a sequestron in which the repressor is CasE, which is encoded by a nucleic acid sequence that comprises target sequences for miRNA, such as miR-122. When miR-122 is present, the output molecule mKate2 is produced, whereas when miR-122 is absent, CasE is produced, which cleaves the mKate2 transcript at a CasE recognition sequence and inhibits production of mKate2. NeonGreen is produced independently of miR-122 presence as a control protein. **FIG. 1C** shows the amount of mKate2 signal observed in the absence (left bar of each group) or presence (right bar of each group) of miR-122.

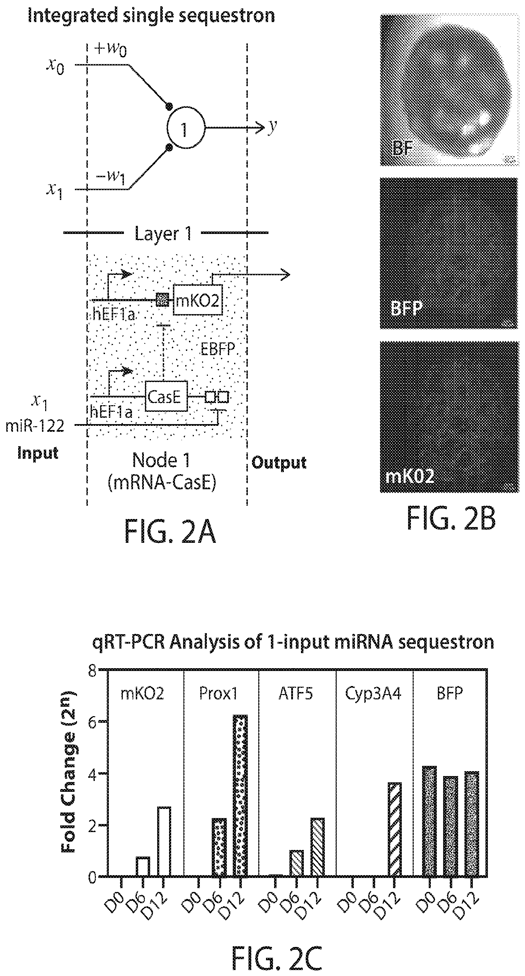

**FIGs. 2A-2C** show an example of a sequestron with one input and four output molecules. **FIG. 2A** shows the circuit schematic (top) and regulatory design (bottom) of a sequestron (integrated single sequestron) in which a sensor circuit encodes CasE under the control of hEFla promoter, and a signal circuit encodes mKO2 under the control of hEFla promoter. The signal circuit contains a CasE recognition sequence upstream of the sequence encoding mKO2, and the sensor circuit contains a target sequence for miR-122 downstream of the sequence encoding CasE, such that mKO2 is expressed only in the presence of miR-122. **FIG. 2B** shows fluorescence microscopy of a liver organoid in which the sequestron is integrated, with mKO2 being expressed in cells containing miR-122 and BFP being expressed in all cells as a control protein. BF = bright field. BFP = blue fluorescent protein. **FIG. 2C** shows quantification by qRT-PCR of expression genes encoded by the sequestron (*mKO2, Prox1, ATF5, Cyp3A4*)*.* BFP is constitutively expressed as a control protein.

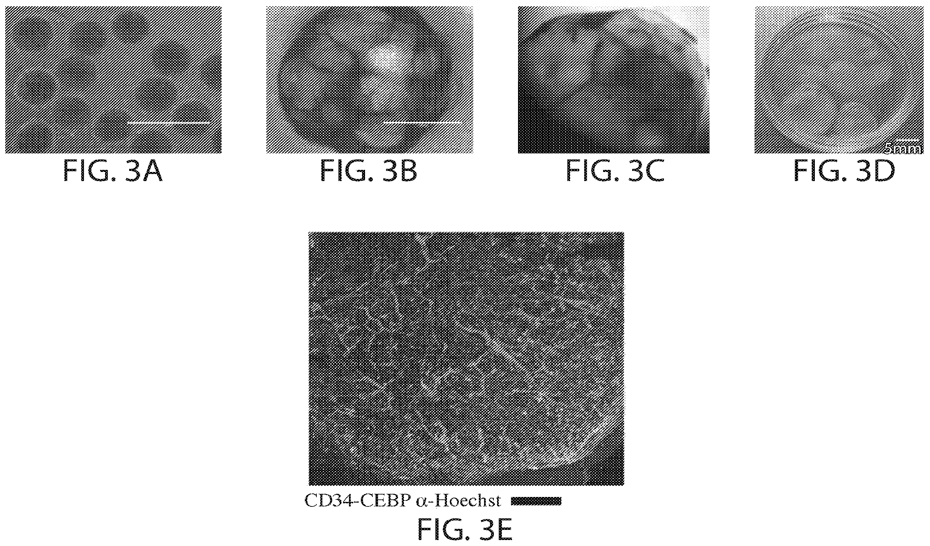

**FIGs. 3A-3E** show the growth and development of 3D liver organoids induced by expression of GATA6. **FIGs. 3A-3B** show the initial formation of 3D GATA6 organoids from days 0-12. **FIGs. 3C-3D** show 3D organoids grown continuously in culture, at day 72. **FIG. 3E** shows vascularized networks (bright branches) comprising CD34⁺ cells co-developing alongside C/EBPα⁺ hepatic-like cells within 3D GATA6 organoids. Scale bars represent 1 mm **(****FIGs. 3A-3C****),** 5 mm **(****FIG. 3D****),** or 0.25 mm **(****FIG. 3E****).**

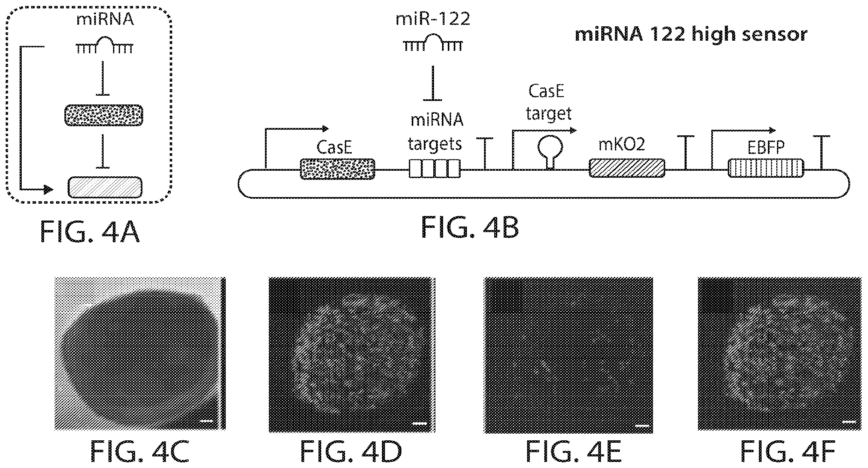

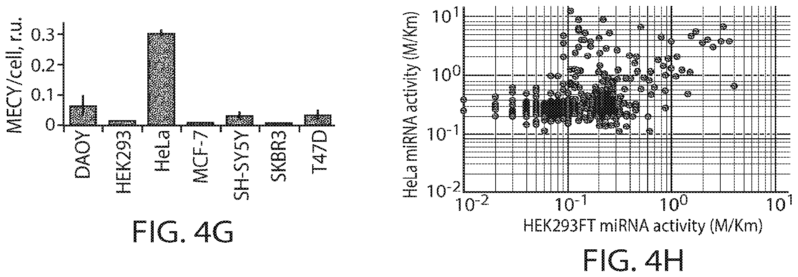

**FIGs. 4A-4H** show the design and validation of sequestrons that produce an output molecule in the presence of an miRNA signal. **FIG. 4A** shows that the presence of an miRNA inhibits a repressor of an output molecule, and thus the presence of the miRNA results in production of the output molecule. **FIG. 4B** shows a detailed design of a sequestron in which the repressor is CasE, which is encoded by a nucleic acid sequence that comprises target sequences for miRNA, such as miR-122. When miR-122 is present, the output molecule mKO2 is produced, whereas when miR-122 is absent, CasE is produced, which inhibits production of mKO2. EBFP is produced independently of miR-122 presence as a control protein. **FIG. 4C** shows bright field microscopy of a liver organoid in which the sequestron has been integrated at day 14 of development. **FIG. 4D** shows fluorescence of BFP. **FIG. 4E** shows fluorescence of mKO2. **FIG. 4F** shows a merged image of the images of **FIGs. 4D-4E****.** **FIG. 4G** shows previous results using a sequestron with 6 inputs for selective gene expression in cancer cells but not in healthy cells (Xie et al. Science. 2011. 333(6047):1307-1311). **FIG. 4H** shows selectivity of miRNA sensors, based on a screen using an miRNA-sensing library that reveals endogenous miRNAs with differential expression in HeLa and HEK293FT cells (Gam et al. Nat Commun. 2018. 9(1):1-12).

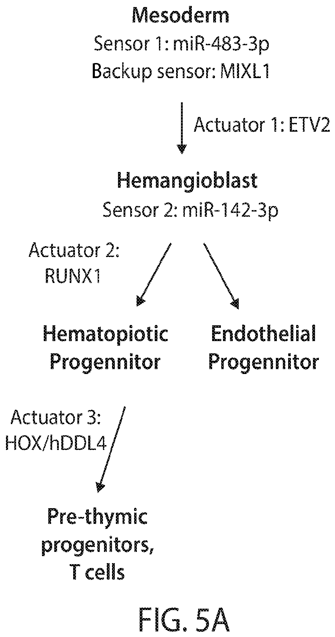

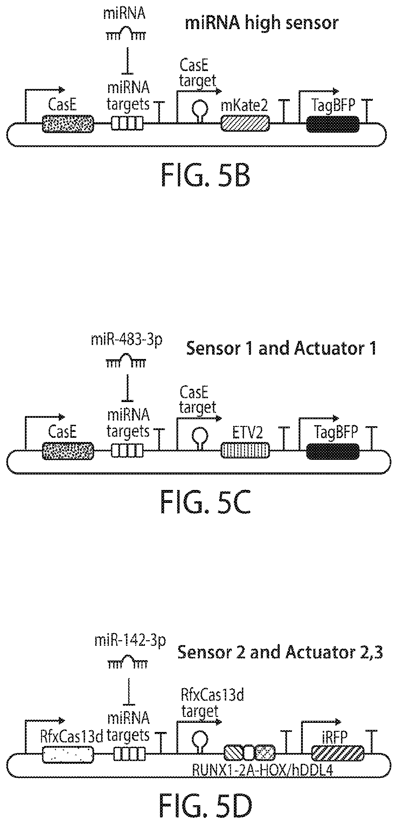

**FIGs. 5A-5D** show a design of multiple sequestrons for expression of different output molecules at different stages of development. **FIG. 5A** shows an overview of the stages of differentiation experienced by developing immune cells, the miRNAs used as input signals for each stage of development, and the actuators expressed at each stage based on the presence of respective miRNAs. **FIG. 5B** shows an overview of the sequestron used to express an output molecule (mKO2) in the presence of an miRNA. **FIG. 5C** shows the sequestron used to express the actuator ETV2 in the presence of miR-483-3p, which is present in mesoderm cells. A constitutive promoter drives transcription of an RNA that encodes CasE and has a target sequence for miR-483-3p, and a second constitutive promoter drives transcription of an RNA that encodes ETV2 and has a CasE recognition sequence, such that ETV2 is translated only when miR-483-3p is present. **FIG. 5D** shows the sequestron used to express the actuator RUNX1 and HOX/hDDL4 in the presence of miR-142-3p, which is present in hemangioblasts. A constitutive promoter drives transcription of an RNA that encodes RfxCas13d and has a target sequence for miR-142-3p, and a second constitutive promoter drives transcription of an RNA that encodes RNX1-2A-HOX/hDDL4 and has a CasE recognition sequence, such that RNX1-2A-HOX/hDDL4 is translated only when miR-142-3p is present. Following translation of the RUNX1-2A-HOX/hDDL4 polypeptide, the 2A motif results in cleavage of the polypeptide and release of separate RUNX1 and HOX/hDDL4 proteins.

### DETAILED DESCRIPTION

### Sequestrons

The present disclosure provides, in some aspects, a sequestron comprising:
(i) a sensor circuit comprising a first constitutive promoter operably linked to a nucleic acid sequence encoding:
   (a) a nucleic acid sequence encoding a repressor; and
   (b) one or more target sequences for a first set of one or more miRNAs; and
(ii) a signal circuit comprising a second constitutive promoter operably linked to a nucleic acid sequence encoding:
   (a) a repressor recognition sequence that is capable of being cleaved by the repressor of (i)(a); and
   (b) a nucleic acid sequence encoding an output molecule.

Sensor circuits of the present disclosure express a repressor only in the presence of one or more inputs. Signal circuits of the present disclosure express an output molecule only in the absence of a repressor encoded by the sensor circuit. Thus, a sensor circuit and signal circuit can be combined to form a sequestron of the present disclosure, which produces an output molecule only in the presence of the inputs that inhibit expression of the repressor. Sensor circuits of the sequestrons disclosed herein comprise a first constitutive promoter operably linked to a nucleic acid sequence encoding a), a nucleic acid sequence encoding a repressor; and b) one or more target sequences for a first set of miRNAs.

In some embodiments, a sequestron regulates expression of an encoded output molecule such that the output molecule is expressed in cells having a particular miRNA profile *(e.g.,* the presence of one or more miRNAs, and/or the absence of one or more different miRNAs). A "microRNA profile," as used herein, refers to the expression levels of one or more microRNAs in a cell or a cell type. The microRNA profile may contain expression levels of microRNAs that have no expression or lower expression (*e.g.,* at least 30% lower), and/or expression levels of microRNAs that express or have higher expression (*e.g.,* at least 30% higher) in a cell or a cell type, compared to another cell or a different cell type, respectively. MicroRNAs that have no expression or lower expression is referred to herein as "microRNA-low" or "miR-low," while microRNAs that express or have high expression is referred to herein as "microRNA-high" or "miR-high."

In part, sequestrons of the present disclosure may detect miRNA by incorporating target sites of the miRNA to be detected into different genetic circuits (*e.g.,* sensor circuit and/or signal circuit). Expression of the microRNA leads to the degradation of mRNAs encoding the molecules that are produced by these circuits (*e.g.,* repressors and/or output molecules), thus leading to different signal output by the sequestron, which may be detected and used for classifying the cell and/or selectively expressing an output molecule to have a biological effect in the cell. Multiple inputs (*e.g.,* microRNAs) can be sensed simultaneously by coupling their detection to different portions of the genetic circuit such that the output molecule is produced only when a certain input profile of miRNAs is detected.

The sequestrons may be used in various applications. In some embodiments, the genetic circuits described herein may be used for the detection of a diseased cell *(e.g.,* a cancer cell). In some embodiments, detection of the diseased cell *(e.g.,* the cancer cell) may be achieved via the expression of a detectable output molecule *(e.g.,* a nucleic acid, a soluble protein, and/or a fluorescent protein) upon detection of a matching microRNA profile. As such, the sequestrons of the present disclosure may be used for diagnosing a disease (*e.g.,* cancer). In some embodiments, detection of the diseased cell *(e.g.,* a cancer cell) may be coupled with the expression of a therapeutic molecule for treating a disease (*e.g.,* cancer). Further, to evaluate the performance of the sequestrons described herein, a large combinatorial library of circuit variants is generated and the performance of each sequestron variant may be evaluated in living cell assays.

In some embodiments of the sequestrons provided herein, a sensor circuit comprises a constitutive promoter operably linked to a nucleic acid sequence encoding a repressor. In some embodiments, a signal circuit comprises a constitutive promoter operably linked to a nucleotide sequence encoding an output molecule. A promoter is a nucleic acid sequence that controls expression of a gene or nucleic acid sequence to which it is operably linked. A promoter is said to be operably linked to a gene if the promoter controls the degree to which the gene is expressed. A promoter may be a constitutive promoter, which results in expression of an operably linked gene at a consistent level. A promoter may be a conditional promoter, which regulates expression of an operably linked gene based on environmental conditions, such as the presence, absence, or amount of a stimulus, such as a small molecule, protein, or nucleic acid. In some embodiments, the first and/or second constitutive promoters are hEFla promoters.

A nucleic acid sequence is said to encode a protein or gene product if a nucleic acid comprising the sequence can be translated by cellular machinery, in the case of an RNA sequence, to produce the protein or gene product. If the nucleic acid sequence is a DNA sequence, then it is said to encode a protein or gene product if the DNA sequence can be transcribed to produce an RNA sequence that can then be translated to produce the protein or gene product.

A repressor, as used herein, refers to a protein or nucleic acid molecule that is capable of inhibiting translation of an RNA. In some embodiments of the sequestrons provided herein, the repressor is an endoribonuclease, an RNAi molecule, or a ribozyme. RNAi molecules are RNA interference molecules (*e.g.* microRNA, miRNA, siRNA, shRNA) that bind to RNA molecules with complementary sequences and, following binding, prevent translation and/or induce degradation of the bound RNA molecule. Ribozymes are nucleic acid enzymes, or nucleic acids with catalytic activity. RNAi molecules, ribozymes, and the use of each in silencing gene expression are familiar to those skilled in the art.

In some embodiments, the repressor is a CRISPR endoribonuclease, and the repressor recognition sequence is a CRISPR endoribonuclease recognition sequence. An endoribonuclease or CRISPR endonuclease, as used herein, refers to a nuclease that cleaves an RNA molecule in a sequence specific manner, *e.g.,* at a target sequence. Sequence-specific endoribonucleases have been described in the art. For example, the *Pyrococcus furiosus* CRISPR-associated endoribonuclease 6 (Cas6) is found to cleave RNA molecules in a sequence-specific manner (Carte et al., Genes & Dev. 2008. 22: 3489-3496). In another example, endoribonucleases that cleave RNA molecules in a sequence-specific manner are engineered, which recognize an 8-nucleotide (nt) RNA sequence and make a single cleavage in the target (Choudhury et al., Nat Commun 3, 1147 (2012). In some embodiments, the endoribonuclease belongs to the CRISPR-associated endoribonuclease. In some embodiments, the endoribonuclease belongs to the CRISPR-associated endoribonuclease 6 (Cas6) family. Cas6 family nucleases from different bacterial species may be used. Non-limiting examples of Cas6 family nucleases include Cas6, Csy4 (also known as Cas6f), Cse3, and CasE. In some embodiments, the endoribonuclease is Cas6, Csy4, CasE, Cse3, LwaCas13a, PspCas13b, RanCas13b, PguCas13b, or RfxCas13d.

A target site, or target sequence, of an miRNA, as used herein, refers to a nucleic acid sequence that is complementary to an miRNA. A first nucleic acid sequence is complementary to a second nucleic acid sequence if a nucleic acid comprising the first sequence binds to a nucleic acid comprising the second sequence, forming a nucleic acid that is at least partially double-stranded through hydrogen bonds between base pairs on the miRNA and target sequence. A first sequence is most complementary to a second sequence when the first sequence comprises a sequence of bases that form canonical Watson-Crick base pairs (*i.e.,* A-U, A-T, C-G) with the target sequence, in reverse order relative to the order of bases in the target sequence. A nucleic acid with this sequence of complementary bases in reverse order is said to have the reverse complement of the target sequence. For example, the reverse complement of the target sequence AAGUCCA is TGGACTT (DNA) or UGGACUU (RNA). An miRNA may still bind to a target sequence even if the sequence of the miRNA differs from the exact reverse complement of the target sequence by one or more nucleotides, provided the sequence of the miRNA is sufficiently similar to the reverse complement of the target sequence. The exact level of sequence identity between the sequence of an miRNA and the reverse complement of the target sequence that is sufficient for an miRNA to bind to a given target sequence will depend on the sequences of the miRNA and target sequence, for example, the nucleotide composition and/or length, as well as the binding conditions (*e.g., in vivo* human physiological conditions). Methods of determining whether an miRNA comprising a given sequence binds to a nucleic acid comprising a target sequence are well known in the art. Following binding of an miRNA to a target sequence, the nucleic acid comprising the target sequence is degraded by cellular machinery of the targeted RNA-directed miRNA degradation (TDMD) pathway.

In some embodiments, a constitutive promoter of a sensor circuit is operably linked to a nucleotide sequence encoding a repressor and one or more target sites for one or more of a first set of miRNAs, or "miRNA-high miRNAs." The placement of target sites for a first set of "miRNA-high" miRNAs on a sensor circuit allows the repressor encoded by the sensor circuit to be downregulated by the presence of any one of the first set of miRNA-high miRNAs, such that the repressor is not translated if any of the first set of miRNA-high miRNAs is present in a cell. Thus, if any of the first set of miRNA-high miRNAs is present in a cell, translational repression of the signal circuit output molecule by the sensor circuit repressor is reduced or abrogated.

In some embodiments of the sequestrons provided herein, the sequestron comprises multiple sensor circuits, each sensor circuit encoding a repressor, with each sensor circuit comprising a target site for an miRNA that is not present on another sensor circuit. In such embodiments, a first miRNA-high miRNA is capable of downregulating repressor expression by a first sensor circuit, but the first miRNA-high miRNA is not capable of downregulating repressor expression by a second sensor circuit, if the second sensor circuit does not encode a target site for the first miRNA-high miRNA. Thus, the use of multiple sensor circuits encoding repressors with target sites for distinct miRNA-high miRNAs allows for the design of sequestrons that require a combination of miRNA-high miRNAs to inhibit repressor translation and consequently allow output molecule expression in the cell.

In some embodiments, a constitutive promoter of a signal circuit is operably linked to a nucleotide sequence encoding an output molecule and one or more target sites for one or more of a second set of miRNAs, and the second set of miRNAs does not contain any miRNAs of the first set of miRNAs (*e.g.,* the first set of miRNAs comprises "miRNA-high" miRNAs, and the second set of miRNAs comprises "miRNA-low" miRNAs). Placement of target sites for a second set of "miRNA-low" miRNAs on a signal circuit allows for inhibition of output molecule translation if any of the miRNA-low miRNAs are present in a cell, even when one or more required miRNA-high miRNAs are present and prevent repressor-mediated translational control. In some embodiments, the sequestron comprises multiple signal circuits, each signal circuit encoding an output molecule, with each signal circuit comprising a target site for an miRNA that is not present on a sensor circuit or another signal circuit. In such embodiments, a first miRNA-low miRNA is capable of downregulating output molecule expression by a first signal circuit, but the first miRNA-low miRNA is not capable of downregulating output molecule expression by a second signal circuit, if the second signal circuit does not encode a target site for the first miRNA-low miRNA. Thus, the use of multiple signal circuits encoding repressors with target sites for distinct miRNA-low miRNAs allows for the design of sequestrons that require a combination of miRNA-low miRNAs to prevent output molecule expression, allowing output molecule expression unless a particular combination of miRNA-low miRNAs is present in the cell.

In some embodiments, the presence or absence of an miRNA is an miRNA biomarker signature for a specific cell type in a specific stage of development. Non-limiting examples of the tissues are lung tissue, skin tissue, breast tissue, connective tissue, brain tissue, gastrointestinal tissue, heart tissue, kidney tissue. Non-limiting examples for specific cell types are epithelial cells, endothelial cells, fibroblasts, immune cells. In some embodiments, the presence or absence of an miRNA in a cell is an miRNA biomarker signature for the type of the cell. In some embodiments, the cell is an endoderm, mesoderm, or ectoderm cell. In some embodiments, the cell is a stem cell. In some embodiments, the stem cell is multipotent, pluripotent, or totipotent. In some embodiments, the stem cell is an embryonic stem cell or an induced pluripotent stem cell. In some embodiments, the cell is a hemangioblast. In some embodiments, the cell is a hematopoietic progenitor cell or endothelial progenitor cell. In some embodiments, the cell is a T cell precursor. In some embodiments, the cell is a hematopoietic stem cell. In some embodiments, the cell is an immune cell. In some embodiments, the immune cell is a T cell, B cell, NK cell, monocyte, macrophage, dendritic cell, neutrophil, eosinophil, or basophil. In some embodiments, the T cell is a CD4⁺ T cell or a CD8⁺ T cell. In some embodiments, the presence or absence of an miRNA is an miRNA biomarker signature for a diseased cell. Non-limiting examples of a diseased cells are neoplastic cells, infected cells, cells harboring genetic mutations, fibro genetic cells. Methods of identifying an miRNA biomarker signature in a specific tissue or cell are known in the art. Information about the sequences, origins, and functions of known miRNAs maybe found in publicly available databases (*e.g*., mirbase.org/, all versions, as described in Kozomara et al., Nucleic Acids Res 2014 42:D68-D73; Kozomara et al., Nucleic Acids Res 2011 39:D152-D157; Griffiths-Jones et al., Nucleic Acids Res 2008 36:D154-D158; Griffiths-Jones et al., Nucleic Acids Res 2006 34:D140-D144; and Griffiths-Jones et al., Nucleic Acids Res 2004 32:D109-D111, including the most recently released version miRBase 21, which contains "high confidence" miRNAs).

Non-limiting examples of miRNAs that are expressed in cells and are able to be detected by the sequestron are: FF4, FF5, hsa-let-7a-2-3p, hsa-let-7a-3p, hsa-let-7a-5p, hsa-let-7b-3p, hsa-let-7b-5p, hsa-let-7c-5p, hsa-let-7d-3p, hsa-let-7d-5p, hsa-let-7e-3p, hsa-let-7e-5p, hsa-let-7f-1-3p, hsa-let-7f-2-3p, hsa-let-7f-5p, hsa-let-7g-3p, hsa-let-7g-5p, hsa-let-7i-5p, hsa-miR-1, hsa-miR-1-3p, hsa-miR-1-5p, hsa-miR-100-3p, hsa-miR-100-5p, hsa-miR-101-3p, hsa-miR-101-5p, hsa-miR-103a-2-5p, hsa-miR-103a-3p, hsa-miR-105-3p, hsa-miR-105-5p, hsa-miR-106a-3p, hsa-miR-106a-5p, hsa-miR-106b-3p, hsa-miR-106b-5p, hsa-miR-107, hsa-miR-10a-3p, hsa-miR-10a-5p, hsa-miR-10b-3p, hsa-miR-10b-5p, hsa-miR-1185-1-3p, hsa-miR-1185-2-3p, hsa-miR-1185-5p, hsa-miR-122-3p, hsa-miR-122a-5p, hsa-miR-1249-3p, hsa-miR-1249-5p, hsa-miR-124a-3p, hsa-miR-125a-3p, hsa-miR-125a-5p, hsa-miR-125b-1-3p, hsa-miR-125b-2-3p, hsa-miR-125b-5p, hsa-miR-126-3p, hsa-miR-126-5p, hsa-miR-127-3p, hsa-miR-1271-3p, hsa-miR-1271-5p, hsa-miR-1278, hsa-miR-128-1-5p, hsa-miR-128-2-5p, hsa-miR-128-3p, hsa-miR-1285-3p, hsa-miR-1285-5p, hsa-miR-1287-3p, hsa-miR-1287-5p, hsa-miR-129-1-3p, hsa-miR-129-2-3p, hsa-miR-129-5p, hsa-miR-1296-3p, hsa-miR-1296-5p, hsa-miR-1304-3p, hsa-miR-1304-5p, hsa-miR-1306-3p, hsa-miR-1306-5p, hsa-miR-1307-3p, hsa-miR-1307-5p, hsa-miR-130a-3p, hsa-miR-130b-3p, hsa-miR-130b-5p, hsa-miR-132-3p, hsa-miR-132-5p, hsa-miR-133a-3p, hsa-miR-133a-5p, hsa-miR-133b, hsa-miR-134-3p, hsa-miR-134-5p, hsa-miR-135a-3p, hsa-miR-135a-5p, hsa-miR-135b-3p, hsa-miR-135b-5p, hsa-miR-136-3p, hsa-miR-136-5p, hsa-miR-138-1-3p, hsa-miR-138-5p, hsa-miR-139-3p, hsa-miR-139-5p, hsa-miR-140-3p, hsa-miR-140-5p, hsa-miR-141-3p, hsa-miR-141-5p, hsa-miR-142-3p, hsa-miR-142-5p, hsa-miR-143-3p, hsa-miR-143-5p, hsa-miR-144-3p, hsa-miR-144-5p, hsa-miR-145-5p, hsa-miR-146a-3p, hsa-miR-146a-5p, hsa-miR-147a, hsa-miR-148a-3p, hsa-miR-148a-5p, hsa-miR-148b-3p, hsa-miR-148b-5p, hsa-miR-149-3p, hsa-miR-144-3p, hsa-miR-150-3p, hsa-miR-150-5p, hsa-miR-151a-3p, hsa-miR-151a-5p, hsa-miR-152-3p, hsa-miR-152-5p, hsa-miR-154-3p, hsa-miR-154-5p, hsa-miR-155-3p, hsa-miR-155-5p, hsa-miR-15a-3p, hsa-miR-15a-5p, hsa-miR-15b-3p, hsa-miR-15b-5p, hsa-miR-16-1-3p, hsa-miR-16-2-3p, hsa-miR-16-5p, hsa-miR-17-3p, hsa-miR-17-5p, hsa-miR-181a-3p, hsa-miR-181a-5p, hsa-miR-181b-2-3p, hsa-miR-181b-5p, hsa-miR-181c-5p, hsa-miR-181d-3p, hsa-miR-181d-5p, hsa-miR-182-3p, hsa-miR-182-5p, hsa-miR-183-3p, hsa-miR-183-5p, hsa-miR-185-3p, hsa-miR-185-5p, hsa-miR-186-3p, hsa-miR-186-5p, hsa-miR-188-3p, hsa-miR-188-5p, hsa-miR-18a-3p, hsa-miR-18a-5p, hsa-miR-18b-5p, hsa-miR-1908-3p, hsa-miR-1908-5p, hsa-miR-190a-3p, hsa-miR-190a-5p, hsa-miR-191-3p, hsa-miR-191-5p, hsa-miR-1910-3p, hsa-miR-1910-5p, hsa-miR-192-3p, hsa-miR-192-5p, hsa-miR-193a-3p, hsa-miR-193a-5p, hsa-miR-193b-3p, hsa-miR-193b-5p, hsa-miR-194-3p, hsa-miR-194-5p, hsa-miR-195-3p, hsa-miR-195-5p, hsa-miR-196a-3p, hsa-miR-196a-5p, hsa-miR-196b-3p, hsa-miR-196b-5p, hsa-miR-197-3p, hsa-miR-197-5p, hsa-miR-199a-3p, hsa-miR-199a-5p, hsa-miR-199b-3p, hsa-miR-199b-5p, hsa-miR-19a-3p, hsa-miR-19a-5p, hsa-miR-19b-1-5p, hsa-miR-19b-2-5p, hsa-miR-19b-3p, hsa-miR-200a-3p, hsa-miR-200a-5p, hsa-miR-200b-3p, hsa-miR-200b-5p, hsa-miR-200c-3p, hsa-miR-200c-5p, hsa-miR-202-3p, hsa-miR-202-5p, hsa-miR-203a-3p, hsa-miR-203a-5p, hsa-miR-204-5p, hsa-miR-208b-3p, hsa-miR-208b-5p, hsa-miR-20a-3p, hsa-miR-20a-5p, hsa-miR-20b-3p, hsa-miR-20b-5p, hsa-miR-21-5p, hsa-miR-210-3p, hsa-miR-210-5p, hsa-miR-211-3p, hsa-miR-211-5p, hsa-miR-2116-3p, hsa-miR-2116-5p, hsa-miR-212-3p, hsa-miR-214-3p, hsa-miR-215-5p, hsa-miR-217, JG_miR-218-1-3p, hsa-miR-218-5p, hsa-miR-219a-1-3p, hsa-miR-219a-2-3p, hsa-miR-219a-5p, hsa-miR-219b-3p, hsa-miR-219b-5p, hsa-miR-22-3p, hsa-miR-22-5p, hsa-miR-221-3p, hsa-miR-221-5p, hsa-miR-222-3p, hsa-miR-222-5p, hsa-miR-223-3p, hsa-miR-223-5p, hsa-miR-23a-3p, hsa-miR-23a-5p, hsa-miR-23b-3p, hsa-miR-24-1-5p, hsa-miR-25-3p, hsa-miR-25-5p, hsa-miR-26a-1-3p, hsa-miR-26a-2-3p, hsa-miR-26a-5p, hsa-miR-26b-5p, hsa-miR-27a-3p, hsa-miR-27a-5p, hsa-miR-27b-3p, hsa-miR-27b-5p, hsa-miR-28-3p, hsa-miR-28-5p, hsa-miR-296-3p, hsa-miR-296-5p, hsa-miR-299-3p, hsa-miR-299-5p, hsa-miR-29a-3p, hsa-miR-29a-5p, hsa-miR-29b-1-5p, hsa-miR-29b-3p, hsa-miR-29c-3p, hsa-miR-301a-3p, hsa-miR-301a-5p, hsa-miR-301b-3p, hsa-miR-301b-5p, hsa-miR-302a-3p, hsa-miR-302a-5p, hsa-miR-302b-5p, hsa-miR-302c-3p, hsa-miR-302c-5p, hsa-miR-3065-3p, hsa-miR-3065-5p, hsa-miR-3074-3p, hsa-miR-3074-5p, hsa-miR-30a-3p, hsa-miR-30a-5p, hsa-miR-30b-3p, hsa-miR-30b-5p, hsa-miR-30c-1-3p, hsa-miR-30c-2-3p, hsa-miR-30c-5p, hsa-miR-30d-3p, hsa-miR-30d-5p, hsa-miR-30e-3p, hsa-miR-30e-5p, hsa-miR-31-3p, hsa-miR-31-5p, hsa-miR-3130-3p, hsa-miR-3130-5p, hsa-miR-3140-3p, hsa-miR-3140-5p, hsa-miR-3144-3p, hsa-miR-3144-5p, hsa-miR-3158-3p, hsa-miR-3158-5p, hsa-miR-32-3p, hsa-miR-32-5p, hsa-miR-320a, hsa-miR-323a-3p, hsa-miR-323a-5p, hsa-miR-324-3p, hsa-miR-324-5p, hsa-miR-326, hsa-miR-328-3p, hsa-miR-328-5p, hsa-miR-329-3p, hsa-miR-329-5p, hsa-miR-330-3p, hsa-miR-330-5p, hsa-miR-331-3p, hsa-miR-331-5p, hsa-miR-335-3p, hsa-miR-335-5p, hsa-miR-337-3p, hsa-miR-337-5p, hsa-miR-338-3p, hsa-miR-338-5p, hsa-miR-339-3p, hsa-miR-339-5p, hsa-miR-33a-3p, hsa-miR-33a-5p, hsa-miR-33b-3p, hsa-miR-33b-5p, hsa-miR-340-3p, hsa-miR-340-5p, hsa-miR-342-3p, hsa-miR-342-5p, hsa-miR-345-3p, hsa-miR-345-5p, hsa-miR-34a-3p, hsa-miR-34a-5p, hsa-miR-34b-3p, hsa-miR-34b-5p, hsa-miR-34c-3p, hsa-miR-34c-5p, hsa-miR-3605-3p, hsa-miR-3605-5p, hsa-miR-361-3p, hsa-miR-361-5p, hsa-miR-3613-3p, hsa-miR-3613-5p, hsa-miR-3614-3p, hsa-miR-3614-5p, hsa-miR-362-3p, hsa-miR-362-5p, hsa-miR-363-3p, hsa-miR-363-5p, hsa-miR-365a-3p, hsa-miR-365a-5p, hsa-miR-365b-3p, hsa-miR-365b-5p, hsa-miR-369-3p, hsa-miR-369-5p, hsa-miR-370-3p, hsa-miR-370-5p, hsa-miR-374a-3p, hsa-miR-374a-5p, hsa-miR-374b-3p, hsa-miR-374b-5p, hsa-miR-375, hsa-miR-376a-2-5p, hsa-miR-376a-3p, hsa-miR-376a-5p, hsa-miR-376c-3p, hsa-miR-376c-5p, hsa-miR-377-3p, hsa-miR-377-5p, hsa-miR-378a-3p, hsa-miR-378a-5p, hsa-miR-379-3p, hsa-miR-379-5p, hsa-miR-381-3p, hsa-miR-381-5p, hsa-miR-382-3p, hsa-miR-382-5p, hsa-miR-409-3p, hsa-miR-409-5p, hsa-miR-411-3p, hsa-miR-411-5p, hsa-miR-412-3p, hsa-miR-421, hsa-miR-423-3p, hsa-miR-423-5p, hsa-miR-424-3p, hsa-miR-424-5p, hsa-miR-425-3p, hsa-miR-425-5p, hsa-miR-431-3p, hsa-miR-431-5p, hsa-miR-432-5p, hsa-miR-433-3p, hsa-miR-433-5p, hsa-miR-449a, hsa-miR-449b-5p, hsa-miR-450a-1-3p, hsa-miR-450a-2-3p, hsa-miR-450a-5p, hsa-miR-450b-3p, hsa-miR-450b-5p, hsa-miR-451a, hsa-miR-452-3p, hsa-miR-4524a-3p, hsa-miR-4524a-5p, hsa-miR-4536-3p, hsa-miR-4536-5p, hsa-miR-454-3p, hsa-miR-454-5p, hsa-miR-4707-3p, hsa-miR-4707-5p, hsa-miR-4755-3p, hsa-miR-4755-5p, hsa-miR-4787-3p, hsa-miR-4787-5p, hsa-miR-483-3p, hsa-miR-483-5p, hsa-miR-484, hsa-miR-485-3p, hsa-miR-485-5p, hsa-miR-487b-3p, hsa-miR-487b-5p, hsa-miR-488-3p, hsa-miR-488-5p, hsa-miR-489-3p, hsa-miR-490-3p, hsa-miR-490-5p, hsa-miR-491-3p, hsa-miR-491-5p, hsa-miR-493-3p, hsa-miR-493-5p, hsa-miR-494-3p, hsa-miR-494-5p, hsa-miR-495-3p, hsa-miR-495-5p, hsa-miR-497-3p, hsa-miR-497-5p, hsa-miR-498, hsa-miR-5001-3p, hsa-miR-5001-5p, hsa-miR-500a-3p, hsa-miR-500a-5p, hsa-miR-5010-3p, hsa-miR-5010-5p, hsa-miR-503-3p, hsa-miR-503-5p, hsa-miR-504-3p, hsa-miR-504-5p, hsa-miR-505-3p, hsa-miR-505-5p, hsa-miR-506-3p, hsa-miR-506-5p, hsa-miR-508-3p, hsa-miR-508-5p, hsa-miR-509-3-5p, hsa-miR-509-3p, hsa-miR-509-5p, hsa-miR-510-3p, hsa-miR-510-5p, hsa-miR-512-5p, hsa-miR-513c-3p, hsa-miR-513c-5p, hsa-miR-514a-3p, hsa-miR-514a-5p, hsa-miR-514b-3p, hsa-miR-514b-5p, hsa-miR-516b-5p, hsa-miR-518c-3p, hsa-miR-518f-3p, hsa-miR-5196-3p, hsa-miR-5196-5p, hsa-miR-519a-3p, hsa-miR-519a-5p, hsa-miR-519c-3p, hsa-miR-519e-3p, hsa-miR-520c-3p, hsa-miR-520f-3p, hsa-miR-520g-3p, hsa-miR-520h, hsa-miR-522-3p, hsa-miR-525-5p, hsa-miR-526b-5p, hsa-miR-532-3p, hsa-miR-532-5p, hsa-miR-539-3p, hsa-miR-539-5p, hsa-miR-542-3p, hsa-miR-542-5p, hsa-miR-543, hsa-miR-545-3p, hsa-miR-545-5p, hsa-miR-548a-3p, hsa-miR-548a-5p, hsa-miR-548ar-3p, hsa-miR-548ar-5p, hsa-miR-548b-3p, hsa-miR-548d-3p, hsa-miR-548d-5p, hsa-miR-548e-3p, hsa-miR-548e-5p, hsa-miR-548h-3p, hsa-miR-548h-5p, hsa-miR-548j-3p, hsa-miR-548j-5p, hsa-miR-548o-3p, hsa-miR-548o-5p, hsa-miR-548v, hsa-miR-551b-3p, hsa-miR-551b-5p, hsa-miR-552-3p, hsa-miR-556-3p, hsa-miR-556-5p, hsa-miR-561-3p, hsa-miR-561-5p, hsa-miR-562, hsa-miR-567, hsa-miR-569, hsa-miR-570-3p, hsa-miR-570-5p, hsa-miR-571, hsa-miR-574-3p, hsa-miR-574-5p, hsa-miR-576-3p, hsa-miR-576-5p, hsa-miR-577, hsa-miR-579-3p, hsa-miR-579-5p, hsa-miR-582-3p, hsa-miR-582-5p, hsa-miR-584-3p, hsa-miR-584-5p, hsa-miR-589-3p, hsa-miR-589-5p, hsa-miR-590-3p, hsa-miR-590-5p, hsa-miR-595, hsa-miR-606, hsa-miR-607, hsa-miR-610, hsa-miR-615-3p, hsa-miR-615-5p, hsa-miR-616-3p, hsa-miR-616-5p, hsa-miR-617, hsa-miR-619-5p, hsa-miR-624-3p, hsa-miR-624-5p, hsa-miR-625-3p, hsa-miR-625-5p, hsa-miR-627-3p, hsa-miR-627-5p, hsa-miR-628-3p, hsa-miR-628-5p, hsa-miR-629-3p, hsa-miR-629-5p, hsa-miR-630, hsa-miR-633, hsa-miR-634, hsa-miR-635, hsa-miR-636, hsa-miR-640, hsa-miR-642a-3p, hsa-miR-642a-5p, hsa-miR-643, hsa-miR-645, hsa-miR-648, hsa-miR-6503-3p, hsa-miR-6503-5p, hsa-miR-651-3p, hsa-miR-651-5p, hsa-miR-6511a-3p, hsa-miR-6511a-5p, hsa-miR-652-3p, hsa-miR-652-5p, hsa-miR-653-5p, hsa-miR-654-3p, hsa-miR-654-5p, hsa-miR-657, hsa-miR-659-3p, hsa-miR-660-3p, hsa-miR-660-5p, hsa-miR-664b-3p, hsa-miR-664b-5p, hsa-miR-671-3p, hsa-miR-671-5p, hsa-miR-675-3p, hsa-miR-675-5p, hsa-miR-7-1-3p, hsa-miR-7-5p, hsa-miR-708-3p, hsa-miR-708-5p, hsa-miR-744-3p, hsa-miR-744-5p, hsa-miR-758-3p, hsa-miR-758-5p, hsa-miR-765, hsa-miR-766-3p, hsa-miR-766-5p, hsa-miR-767-3p, hsa-miR-767-5p, hsa-miR-769-3p, hsa-miR-769-5p, hsa-miR-802, hsa-miR-873-3p, hsa-miR-873-5p, hsa-miR-874-3p, hsa-miR-874-5p, hsa-miR-876-3p, hsa-miR-876-5p, hsa-miR-885-3p, hsa-miR-885-5p, hsa-miR-887-3p, hsa-miR-887-5p, hsa-miR-9-3p, hsa-miR-9-5p, hsa-miR-92a-1-5p, hsa-miR-92a-2-5p, hsa-miR-92a-3p, hsa-miR-92b-3p, hsa-miR-92b-5p, hsa-miR-93-3p, hsa-miR-93-5p, hsa-miR-941, hsa-miR-942-3p, hsa-miR-942-5p, hsa-miR-96-3p, hsa-miR-96-5p, hsa-miR-98-3p, hsa-miR-98-5p, hsa-miR-99a-3p, hsa-miR-99a-5p, hsa-miR-99b-3p, and hsa-miR-99b-5p.

Signal circuits of the sequestrons disclosed herein comprise a second constitutive promoter operably linked to a nucleic acid sequence encoding a), a repressor recognition sequence; and b) an output molecule.

A repressor recognition sequence, as used herein, refers to a nucleic acid sequence that is capable of being recognized and bound by a repressor. Binding of a repressor to a repressor recognition sequence refers to association between nucleic acid sequences of the repressor and repressor recognition sequence, if the repressor is an RNAi molecule or ribozyme, or non-covalent association between the repressor and nucleic acid comprising the repressor recognition sequence, if the repressor is an endoribonuclease. If the repressor is an RNAi molecule, the repressor recognition sequence may comprise a nucleic acid sequence that is complementary to a sequence of the RNAi molecule. If the repressor is a ribozyme, the repressor recognition sequence may comprise a nucleic acid sequence that is complementary to a sequence of the RNAi molecule. If the repressor is an endoribonuclease, the repressor recognition sequence may be a nucleic acid sequence that is capable of being cleaved by the endoribonuclease. Repressor recognition sequences that are capable of being cleaved by given endonucleases are known in the art (see, *e.g.,* DiAndreth et al. bioRxiv. 2019. doi: 10.1101/2019.12.15.867150). Following binding of the repressor to a repressor recognition sequence, the bound nucleic acid is degraded by cellular RNA interference machinery, in the case of an RNAi molecule, or cleaved, in the case of a ribozyme or endoribonuclease.

An output molecule, as used herein, refers to an RNA molecule or protein that is produced only under desired conditions, such as the presence of one or more of a first set of one or more miRNAs, and optionally the absence of one or more of a second set of one or more miRNAs. Non-limiting examples of output molecules include transcription factors, cytokines, chemokines, miRNAs, surface markers, cell surface receptors, and Toll-like receptors. In some embodiments, the output molecule is a transcription factor. Non-limiting examples of transcription factors include ETV2, RUNX1, Scl, Lyl1, Lmo2, Gata2, Meis1, Erg, Gfilb Hoxa5, Hoxa7, Hoxa10, Ikzf1, and Setbp1. In some embodiments, the output molecule is a cell surface receptor that is a Notch ligand. Non-limiting examples of Notch ligands include Jagged-1, Jagged-2, hDLL1, hDLL2, hDLL3, and hDLL4. In some embodiments, the output molecule is ETV2, RUNX1, and/or hDLL4. In some embodiments, the output molecule is ETV2. In some embodiments, the output molecule is a polypeptide comprising RUNX1 and hDLL4.

In some embodiments, the one or more target sequences for the first set of one or more miRNAs of (i)(b) are downstream from the nucleic acid sequence encoding the repressor of (i)(a). A first sequence is said to be downstream from a second sequence on a nucleic acid if, when reading the sequence in the 5'-to-3' direction, the first sequence occurs after the second sequence.

In some embodiments, the repressor recognition sequence of (ii)(a) is upstream from the nucleic acid sequence encoding the output molecule of (ii)(b). A first sequence is said to be upstream from a second sequence on a nucleic acid if, when reading the sequence in the 5'-to-3' direction, the first sequence occurs before the second sequence.

In some embodiments, the nucleic acid sequence encoded by the signal circuit of (ii) further comprises one or more target sequences for a second set of one or more miRNAs. In some embodiments, the one or more target sequences for the second set of one or more miRNAs of are downstream from the nucleic acid sequence encoding the output molecule. The addition of one or more target sequences for a second set of one or more miRNAs allows expression of the output molecule to be negatively regulated by the second set of miRNAs (e.g., expressed only in the absence of any miRNAs that are complementary to the target sequences of the RNAs encoding the output molecule). Therefore signal circuit comprising one or more target sequences for one miRNA operates as a NOT gate, and a signal circuit comprising one or more target sequences for multiple miRNAs operates as a NOR gate, with the inputs being the presence of miRNAs that are complementary to one or more target sequences on the signal circuit.

In some embodiments, the sequestron comprises a plurality of the signal circuit of (ii). A sequestron comprising a plurality of signal circuits may encode multiple output molecules, with each of the plurality of signal circuits encoding different output molecules. In some embodiments, each of the plurality of signal circuits comprises a different constitutive promoter, such that different output molecules are expressed at different levels.

In some embodiments, each of the plurality of signal circuits comprises target sequences for different miRNAs of the second set of one or more miRNAs. The separation of nucleic acid sequences encoding different molecules onto distinct signal circuits allows each of the output molecules to be positively regulated by the same first set of miRNAs (e.g., expressed only if one or more of the first set of miRNAs is present), but negatively regulated by different miRNAs of the second set (*e.g.,* expressed only in the absence of the miRNAs that are complementary to the target sequences of the RNAs encoding the output molecule).

In some embodiments, the sequestron comprises a plurality of the sensor circuit of (i). In some embodiments, each of the plurality of sensor circuits comprises target sequences for different miRNAs. The separation of target sequences for different miRNAs onto to distinct sensor circuits allows the sequestron to activate expression of the output molecule(s) only when each of the sensor circuits is targeted by miRNAs for degradation. For example, if one sensor circuit comprises a target site for miR-122 and a second sensor circuit comprises a target site for miR-483, then miR-122 or miR-483 alone are insufficient to inhibit expression of the repressor, which will prevent expression of the output molecule. In this case, expression of the repressor will be inhibited, and thus the output molecule(s) will be expressed, only in the presence of miR-122 and miR-483. Therefore, a sequestron comprising a plurality of sensor circuits operates as an AND gate, with the inputs being the presence of miRNAs that are complementary to target sequences on each of the plurality of sensor circuits.

### Compositions comprising sequestrons and methods of use

In some aspects, the present disclosure provides a composition comprising a plurality of the sequestrons provided herein, wherein:
(A) the nucleic acid sequence of (i)(a) of each of the plurality of sequestrons encodes a different repressor;
(B) the repressor recognition sequence of (ii)(a) of each of the plurality of sequestrons comprises a different nucleic acid sequence;
(C) the repressor encoded by the sensor circuit of each of the plurality of sequestrons is capable of cleaving the repressor recognition sequence of the signal circuit of the same sequestron; and
(D) the repressor encoded by the sensor circuit of each sequestron is not capable of cleaving the repressor recognition sequence of a different sequestron. In some embodiments, the present disclosure provides a cell comprising a plurality of the sequestrons provided herein. In a cell comprising multiple sequestrons, the provision of separate sensor circuits, each encoding a different repressor, and separate signal circuits, each encoding a different repressor recognition sequence, allows the expression of output molecules to be regulated by environmental stimuli that may change over time. For example, the sensor circuit of a first sequestron may encode a first endoribonuclease, such as CasE, and comprise a target site for a first miRNA, such as miR-122, while the sensor circuit of a second sequestron may encode a second endoribonuclease, such as RfxCas13d, and comprise a target site for a second miRNA, such as miR-483. A cell containing both sensor circuits, as well as a first signal circuit encoding a CasE recognition sequence and a first output molecule, and a second signal circuit encoding a RfxCas13d recognition sequence, will express the first output molecule only when miR-122 is present, and the second output molecule only when miR-483 is present. Methods of determining whether a repressor is capable of cleaving a repressor recognition sequence are known in the art, and the specificity and cross-reactivity of endoribonucleases recited herein have been evaluated (DiAndreth et al. bioRxiv. 2019. doi: 10.1101/2019.12.15.867150).

In some aspects, the present disclosure provides a composition comprising any of the sequestrons, or a composition comprising a plurality of sequestrons, provided herein. In some embodiments, the composition further comprises a pharmaceutically acceptable excipient. "Acceptable" means that the carrier must be compatible with the active ingredient of the composition (and preferably, capable of stabilizing the active ingredient) and not deleterious to the subject to be treated. Pharmaceutically acceptable excipients (carriers) including buffers, which are well known in the art. See, *e.g*., Remington: The Science and Practice of Pharmacy 20th Ed. (2000) Lippincott Williams and Wilkins, Ed. K. E. Hoover.

The pharmaceutical compositions to be used for in vivo administration must be sterile. This is readily accomplished by, for example, filtration through sterile filtration membranes. The pharmaceutical compositions described herein may be placed into a container having a sterile access port, for example, an intravenous solution bag or vial having a stopper pierceable by a hypodermic injection needle.

In other embodiments, the pharmaceutical compositions described herein can be formulated for intra-muscular injection, intravenous injection, intratumoral injection or subcutaneous injection.

The pharmaceutical compositions described herein to be used in the present methods can comprise pharmaceutically acceptable carriers, buffer agents, excipients, salts, or stabilizers in the form of lyophilized formulations or aqueous solutions. See, *e.g.,* Remington: The Science and Practice of Pharmacy 20th Ed. (2000) Lippincott Williams and Wilkins, Ed. K. E. Hoover). Acceptable carriers, excipients, or stabilizers are nontoxic to recipients at the dosages and concentrations used, and may comprise buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrans; chelating agents such as EDTA; sugars such as sucrose, mannitol, trehalose or sorbitol; salt-forming counter-ions such as sodium; metal complexes (*e.g.,* Zn-protein complexes); and/or non-ionic surfactants such as TWEEN^{™}, PLURONICS^{™} or polyethylene glycol (PEG).

In some examples, the pharmaceutical composition described herein comprises lipid nanoparticles which can be prepared by methods known in the art, such as described in Epstein, et al., Proc. Natl. Acad. Sci. USA 82:3688 (1985); Hwang, et al., Proc. Natl. Acad. Sci. USA 77:4030 (1980); and U.S. Pat. Nos. 4,485,045 and 4,544,545. Liposomes with enhanced circulation time are disclosed in U.S. Pat. No. 5,013,556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

In other examples, the pharmaceutical composition described herein can be formulated in sustained-release format. Suitable examples of sustained-release preparations include semipermeable matrices of solid hydrophobic polymers containing the sequestron, the vector comprising the same, or the cell comprising the same, which matrices are in the form of shaped articles, *e.g*., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and 7 ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), sucrose acetate isobutyrate, and poly-D-(-)-3-hydroxybutyric acid.

Suitable surface-active agents include, in particular, non-ionic agents, such as polyoxyethylenesorbitans *(e.g.,* TWEEN^{™} 20, 40, 60, 80 or 85) and other sorbitans (*e.g.,* SPAN^{™} 20, 40, 60, 80 or 85). Compositions with a surface-active agent will conveniently comprise between 0.05 and 5% surface-active agent, and can be between 0.1 and 2.5%. It will be appreciated that other ingredients may be added, for example mannitol or other pharmaceutically acceptable vehicles, if necessary.

The pharmaceutical compositions described herein can be in unit dosage forms such as tablets, pills, capsules, powders, granules, solutions or suspensions, or suppositories, for oral, parenteral or rectal administration, or administration by inhalation or insufflation.

For preparing solid compositions such as tablets, the principal active ingredient can be mixed with a pharmaceutical carrier, *e.g*., conventional tableting ingredients such as corn starch, lactose, sucrose, sorbitol, talc, stearic acid, magnesium stearate, dicalcium phosphate or gums, and other pharmaceutical diluents, *e.g*., water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

Suitable emulsions may be prepared using commercially available fat emulsions, such as INTRALIPID^{™}, LIPOSYN^{™}, INFONUTROL^{™}, LIPOFUNDIN^{™} and LIPIPHYSAN^{™}. The active ingredient may be either dissolved in a pre-mixed emulsion composition or alternatively it may be dissolved in an oil (*e.g.,* soybean oil, safflower oil, cottonseed oil, sesame oil, corn oil or almond oil) and an emulsion formed upon mixing with a phospholipid *(e.g.,* egg phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion can comprise fat droplets having a suitable size and can have a pH in the range of 5.5 to 8.0.

Pharmaceutical compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as set out above. In some embodiments, the compositions are administered by the oral or nasal respiratory route for local or systemic effect.

Compositions in preferably sterile pharmaceutically acceptable solvents may be nebulized by use of gases. Nebulized solutions may be breathed directly from the nebulizing device or the nebulizing device may be attached to a face mask, tent or intermittent positive pressure breathing machine. Solution, suspension or powder compositions may be administered, preferably orally or nasally, from devices which deliver the formulation in an appropriate manner.

Also provided herein are nucleic acid(s) and vector(s) comprising the sequestrons described herein. Each component of the sequestron may be included in one or more (*e.g*., 2, 3 or more) nucleic acid molecules (*e.g.,* vectors) and introduced into a cell. A "nucleic acid" is at least two nucleotides covalently linked together, and in some instances, may contain phosphodiester bonds *(e.g.,* a phosphodiester "backbone"). A nucleic acid may be DNA, both genomic and/or cDNA, RNA or a hybrid, where the nucleic acid contains any combination of deoxyribonucleotides and ribonucleotides (*e.g.,* artificial or natural), and any combination of bases, including uracil, adenine, thymine, cytosine, guanine, inosine, xanthine, hypoxanthine, isocytosine and isoguanine. Nucleic acids of the present disclosure may be produced using standard molecular biology methods (see, *e.g.,* Green and Sambrook, Molecular Cloning, A Laboratory Manual, 2012, Cold Spring Harbor Press).

In some embodiments, nucleic acids are produced using GIBSON ASSEMBLY^{®} Cloning (see, *e.g*., Gibson, D.G. et al. Nature Methods, 343-345, 2009; and Gibson, D.G. et al. Nature Methods, 901-903, 2010). GIBSON ASSEMBLY^{®} typically uses three enzymatic activities in a single-tube reaction: 5' exonuclease, the 3' extension activity of a DNA polymerase and DNA ligase activity. The 5' exonuclease activity chews back the 5' end sequences and exposes the complementary sequence for annealing. The polymerase activity then fills in the gaps on the annealed regions. A DNA ligase then seals the nick and covalently links the DNA fragments together. The overlapping sequence of adjoining fragments is much longer than those used in Golden Gate Assembly, and therefore results in a higher percentage of correct assemblies.

In some aspects, the present disclosure provides methods comprising delivering any of the sequestrons or compositions comprising sequestrons to a cell, and optionally detecting an output molecule. In some embodiments, the sequestron is delivered to a cell by one or more vectors. A "vector" refers to a nucleic acid *(e.g.,* DNA) used as a vehicle to artificially carry genetic material (*e.g.,* an engineered nucleic acid) into a cell where, for example, it can be replicated and/or expressed. In some embodiments, a vector is an episomal vector (see, *e.g.,* Van Craenenbroeck K. et al. Eur. J. Biochem. 267, 5665, 2000). A non-limiting example of a vector is a plasmid, RNA replicons, viral vectors (*e.g.,* rAAV, lentivirus). Plasmids are double-stranded generally circular DNA sequences that are capable of automatically replicating in a host cell. Plasmid vectors typically contain an origin of replication that allows for semi-independent replication of the plasmid in the host and also the transgene insert. Plasmids may have more features, including, for example, a "multiple cloning site," which includes nucleotide overhangs for insertion of a nucleic acid insert, and multiple restriction enzyme consensus sites to either side of the insert. Another non-limiting example of a vector is a viral vector (*e.g.,* retrovirus, adenovirus, adeno-associated virus, helper-dependent adenovirus systems, hybrid adenovirus systems, herpes simplex virus, pox virus, lentivirus, Epstein-Barr virus). In some embodiments, the viral vector is derived from an adeno-associated virus (AAV). In some embodiments, the viral vector is derived from a herpes simplex virus (HSV).

The nucleic acids or vectors containing the sensor and/or signal circuits of the sequestron may be delivered to a cell by any methods known in the art for delivering nucleic acids. For example, for delivering nucleic acids to a prokaryotic cell, the methods include, without limitation, transformation, transduction, conjugation, and electroporation. For delivering nucleic acids to a eukaryotic cell, methods include, without limitation, transfection, electroporation, and using viral vectors. In some embodiments, the sensor circuit of the sequestron and the signal circuit of the sequestron are delivered to the cell by different nucleic acids or vectors. In some embodiments, there are different copy numbers of the sensor circuit and the signal circuit. In some embodiments, the ratio between the sensor circuit and the signal circuit is proportional. Proportional delivery of the sensor circuit and the signal circuit of the sequestron means they are delivered at a ratio. In some embodiments, the ration between the nucleic acids or vectors carrying the sensor circuit of the sequestron and the nucleic acids or vectors carrying the signal circuit is 1:1, 1:2, 1:3, 1:4, 1:5, 1:6, 1:7, 1:8, 1:9, 1:10, 2:3, 2:5, 2:7, 2:9, 3:4, 3:5, 3:7, 3:8, 3:10, 4:5, 4:7, 4:9, 4:10, 5:6, 5:7, 5:8, 5:9, 6:7, 7:8, 7:9, 7:10, 8:9, 9:10, 10:1, 9:1, 8:1, 7:1, 6:1, 5:1, 4:1, 3:1, 2:1, 3:2, 5:2, 7:2, 9:2, 4:3, 5:3, 7:3, 8:3, 10:3, 5:4, 7:4, 9:4, 10:4, 6:5, 7:5, 8:5, 9:5, 7:6, 8:7, 9:7, 10:7, 9:8, or 10:9.

Detecting an output molecule, as used herein, refers to measuring the amount or presence of the output molecule present in or produced by a sequestron comprising a nucleic acid sequence encoding the output molecule. Methods of measuring the amount or presence of an output molecule are well known in the art, with non-limiting methods of measurement including ELISA, PCR, qRT-PCR, fluorescence-activated cell sorting (FACS), microscopy, and fluorescent microscopy.

Also provided herein are cells comprising the sequestron or the vectors encoding the same as described herein. A "cell" is the basic structural and functional unit of all known independently living organisms. It is the smallest unit of life that is classified as a living thing. Some organisms, such as most bacteria, are unicellular (consist of a single cell). Other organisms, such as humans, are multicellular.

In some embodiments, the present disclosure provides methods of maintaining a cell *(e.g.,* in culture) comprising any of the sequestrons provided herein. Methods of maintaining cells in culture are known in the art, and include incubating cells in the presence of a medium and environmental conditions (*e.g.,* temperature, humidity, atmospheric gas concentrations) suitable for maintaining cellular metabolism and keeping cells alive. Suitable media and environmental conditions for maintaining cells in culture may vary depending on cell type, physiology, and/or disease state, but may be determined by observing cellular behavior under a given set of conditions and determining whether cells maintain metabolism and remain living under such conditions.

In some embodiments, the methods provided herein comprise detecting the output molecule encoded by the sequestron in a cell. In some embodiments, the method further comprises classifying the cell on the basis of output molecule expression, with expression classifying the cell in one manner, and lack of expression classifying the cell differently. For example, output molecule expression may classify the cell as belonging to a developmental stage, while lack of output molecule expression classifies the cell as belonging to a different developmental stage. As another example, output molecule expression may classify the cell as being a diseased cell, whereas lack of output molecule expression classifies the cell as not being a diseased cell.

In some embodiments, a cell for use in accordance with the present disclosure is a prokaryotic cell, which may comprise a cell envelope and a cytoplasmic region that contains the cell genome (DNA) and ribosomes and various sorts of inclusions. In some embodiments, the cell is a bacterial cell. As used herein, the term "bacteria" encompasses all variants of bacteria, for example, prokaryotic organisms and cyanobacteria. Bacteria are small (typical linear dimensions of around 1 micron), non-compartmentalized, with circular DNA and ribosomes of 70S. The term bacteria also includes bacterial subdivisions of Eubacteria and Archaebacteria. Eubacteria can be further subdivided into gram-positive and gram-negative Eubacteria, which depend upon a difference in cell wall structure. Also included herein are those classified based on gross morphology alone *(e.g.,* cocci, bacilli). In some embodiments, the bacterial cells are gram-negative cells, and in some embodiments, the bacterial cells are gram-positive cells. Examples of bacterial cells that may be used in accordance with the invention include, without limitation, cells from *Yersinia spp., Escherichia spp., Klebsiella spp., Bordetella spp., Neisseria spp., Aeromonas spp., Francisella spp., Corynebacterium spp., Citrobacter spp., Chlamydia spp., Haemophilus spp., Brucella spp., Mycobacterium spp., Legionella spp., Rhodococcus spp., Pseudomonas spp., Helicobacter spp., Salmonella spp., Vibrio spp., Bacillus spp., Erysipelothrix spp., Salmonella spp.,* and/or *Streptomyces spp.* In some embodiments, the bacterial cells are from *Staphylococcus aureus, Bacillus subtilis, Clostridium butyricum, Brevibacterium lactofermentum, Streptococcus agalactiae, Lactococcus lactis, Leuconostoc lactis, Streptomyces, Actinobacillus actinobycetemcomitans, Bacteroides,* cyanobacteria, *Escherichia coli, Helicobacter pylori, Selnomonas ruminatium, Shigella sonnei, Zymomonas mobilis, Mycoplasma mycoides, Treponema denticola, Bacillus thuringiensis, Staphlococcus lugdunensis, Leuconostoc oenos, Corynebacterium xerosis, Lactobacillus plantarum, Streptococcus faecalis, Bacillus coagulans, Bacillus cereus, Bacillus popillae, Synechocystis* strain PCC6803, *Bacillus liquefaciens, Pyrococcus abyssi, Selenomonas nominantium, Lactobacillus hilgardii, Streptococcus ferus, Lactobacillus pentosus, Bacteroides fragilis, Staphylococcus epidermidis, Zymomonas mobilis, Streptomyces phaechromogenes, Streptomyces ghanaenis, Halobacterium* strain GRB, or *Halobaferax* sp. strain Aa2.2.

In some embodiments, a cell for use in accordance with the present disclosure is a eukaryotic cell, which comprises membrane-bound compartments in which specific metabolic activities take place, such as a nucleus. Examples of eukaryotic cells for use in accordance with the invention include, without limitation, mammalian cells, insect cells, yeast cells (e.g., Saccharomyces cerevisiae) and plant cells. In some embodiments, the eukaryotic cells are from a vertebrate animal. In some embodiments, the cell is a mammalian cell. In some embodiments, the cell is a human cell. In some embodiments, the cell is from a rodent, such as a mouse or a rat. Examples of vertebrate cells for use in accordance with the present disclosure include, without limitation, reproductive cells including sperm, ova and embryonic cells, and non-reproductive cells, immune, kidney, lung, spleen, lymphoid, cardiac, gastric, intestinal, pancreatic, muscle, bone, neural, brain and epithelial cells. Stem cells, including embryonic stem cells or induced pluripotent stem cells, can also be used.

In some embodiments, the cell is a diseased cell. A "diseased cell," as used herein, refers to a cell whose biological functionality is abnormal, compared to a non-diseased (normal) cell. In some embodiments, the diseased cell is a cancer cell.

In some embodiments, the cell is a cell used for recombinant protein production. Non-limiting examples of recombinant protein producing cells are Chinese hamster ovary (CHO) cells, human embryonic kidney (HEK)-293 cells, verda reno (VERO) cells, nonsecreting null (NS0) cells, human embryonic retinal (PER.C6) cells, Sp2/0 cells, baby hamster kidney (BHK) cells, Madin-Darby Canine Kidney (MDCK) cells, Madin-Darby Bovine Kidney (MDBK) cells, and monkey kidney CV1 line transformed by SV40 (COS) cells.

In some aspects, the present disclosure provides methods of treating a disease or disorder, the method comprising delivering any of the sequestrons or compositions comprising sequestrons provided herein to a subject in need thereof, wherein the output molecule is a therapeutic molecule that treats the disease or disorder. In some embodiments, the output molecule is a therapeutic molecule. A "therapeutic molecule" is a molecule that has therapeutic effects on a disease or condition, and may be used to treat a diseases or condition. Therapeutic molecules of the present disclosure may be nucleic acid-based or protein or polypeptide-based.

In some embodiments, nucleic acid-based therapeutic molecule may be an RNA interference (RNAi) molecule *(e.g.,* a microRNA, siRNA, or shRNA) or an nucleic acid enzyme *(e.g.,* a ribozyme). RNAi molecules and there use in silencing gene expression are familiar to those skilled in the art. In some embodiments, the RNAi molecule targets an oncogene. An oncogene is a gene that in certain circumstances can transform a cell into a tumor cell. An oncogene may be a gene encoding a growth factor or mitogen (*e.g.,* c-Sis), a receptor tyrosine kinase (e.g., EGFR, PDGFR, VEGFR, or HER2/neu), a cytoplasmic tyrosine kinase *(e.g.,* Src family kinases, Syk-ZAP-70 family kinases, or BTK family kinases), a cytoplasmic serine/threonine kinase or their regulatory subunits (*e.g.,* Raf kinase or cyclin-dependent kinase), a regulatory GTPase *(e.g.,* Ras), or a transcription factor (*e.g.,* Myc). In some embodiments, the oligonucleotide targets Lipocalin (Lcn2) *(e.g.,* a Lcn2 siRNA). One skilled in the art is familiar with genes that may be targeted for the treatment of cancer.

Non-limiting examples of protein or polypeptide-based therapeutic molecules include enzymes, regulatory proteins (*e.g.,* immuno-regulatory proteins), antigens, antibodies or antibody fragments, and structural proteins. In some embodiments, the protein or polypeptide-based therapeutic molecules are for cancer therapy.

Suitable enzymes (for operably linking to a synthetic promoter) for some embodiments of this disclosure include, for example, oxidoreductases, transferases, polymerases, hydrolases, lyases, synthases, isomerases, and ligases, digestive enzymes (*e.g.,* proteases, lipases, carbohydrases, and nucleases). In some embodiments, the enzyme is selected from the group consisting of lactase, beta-galactosidase, a pancreatic enzyme, an oil-degrading enzyme, mucinase, cellulase, isomaltase, alginase, digestive lipases (*e.g.,* lingual lipase, pancreatic lipase, phospholipase), amylases, cellulases, lysozyme, proteases (*e.g.,* pepsin, trypsin, chymotrypsin, carboxypeptidase, elastase,), esterases (*e.g.* sterol esterase), disaccharidases (*e.g.,* sucrase, lactase, beta-galactosidase, maltase, isomaltase), DNases, and RNases.

Non-limiting examples of antibodies and fragments thereof include: bevacizumab (AVASTIN^{®}), trastuzumab (HERCEPTIN^{®}), alemtuzumab (CAMPATH^{®}, indicated for B cell chronic lymphocytic leukemia,), gemtuzumab (MYLOTARG^{®}, hP67.6, anti-CD33, indicated for leukemia such as acute myeloid leukemia), rituximab (RITUXAN^{®}), tositumomab (BEXXAR^{®}, anti-CD20, indicated for B cell malignancy), MDX-210 (bispecific antibody that binds simultaneously to HER-2/neu oncogene protein product and type I Fc receptors for immunoglobulin G (IgG) (Fc gamma RI)), oregovomab (OVAREX^{®}, indicated for ovarian cancer), edrecolomab (PANOREX^{®}), daclizumab (ZENAPAX^{®}), palivizumab (SYNAGIS^{®}, indicated for respiratory conditions such as RSV infection), ibritumomab tiuxetan (ZEVALIN^{®}, indicated for Non-Hodgkin's lymphoma), cetuximab (ERBITUX^{®}), MDX-447, MDX-22, MDX-220 (anti-TAG-72), IOR-C5, IOR-T6 (anti-CD1), IOR EGF/R3, celogovab (ONCOSCINT^{®} OV103), epratuzumab (LYMPHOCIDE^{®}), pemtumomab (THERAGYN^{®}), Gliomab-H (indicated for brain cancer, melanoma). In some embodiments, the antibody is an antibody that inhibits an immune check point protein, *e.g.,* an anti-PD-1 antibody such as pembrolizumab (KEYTRUDA^{®}) or nivolumab (OPDIVO^{®}), or an anti-CTLA-4 antibody such as ipilimumab (YERVOY^{®}). Other antibodies and antibody fragments may be operably linked to a synthetic promoter, as provided herein.

A regulatory protein may be, in some embodiments, a transcription factor or a immunoregulatory protein. Non-limiting, exemplary transcriptional factors include: those of the NFkB family, such as Rel-A, c-Rel, Rel-B, p50 and p52; those of the AP-1 family, such as Fos, FosB, Fra-1, Fra-2, Jun, JunB and JunD; ATF; CREB; STAT-1, -2, -3, -4, -5 and -6; NFAT-1, -2 and -4; MAF; Thyroid Factor; IRF; Oct-1 and -2; NF-Y; Egr-1; and USF-43, EGR1, Sp1, and E2F1. Other transcription factors may be operably linked to a synthetic promoter, as provided herein.

As used herein, an immunoregulatory protein is a protein that regulates an immune response. Non-limiting examples of immunoregulatory include: antigens, adjuvants (*e.g.,* flagellin, muramyl dipeptide), cytokines including interleukins (*e.g.,* IL-2, IL-7, IL-15 or superagonist/mutant forms of these cytokines), IL-12, IFN-gamma, IFN-alpha, GM-CSF, FLT3-ligand), and immunostimulatory antibodies (*e.g.,* anti-CTLA-4, anti-CD28, anti-CD3, or single chain/antibody fragments of these molecules). Other immunoregulatory proteins may be operably linked to a synthetic promoter, as provided herein.

As used herein, an antigen is a molecule or part of a molecule that is bound by the antigen-binding site of an antibody. In some embodiments, an antigen is a molecule or moiety that, when administered to or expression in the cells of a subject, activates or increases the production of antibodies that specifically bind the antigen. Antigens of pathogens are well known to those of skill in the art and include, but are not limited to parts (coats, capsules, cell walls, flagella, fimbriae, and toxins) of bacteria, viruses, and other microorganisms. Examples of antigens that may be used in accordance with the disclosure include, without limitation, cancer antigens, self-antigens, microbial antigens, allergens and environmental antigens. Other antigens may be operably linked to a synthetic promoter, as provided herein.

In some embodiments, the antigen of the present disclosure is a cancer antigen. A cancer antigen is an antigen that is expressed preferentially by cancer cells (*i.e.,* it is expressed at higher levels in cancer cells than in non-cancer cells) and, in some instances, it is expressed solely by cancer cells. Cancer antigens may be expressed within a cancer cell or on the surface of the cancer cell. Cancer antigens that may be used in accordance with the disclosure include, without limitation, MART-1/Melan-A, gp100, adenosine deaminase-binding protein (ADAbp), FAP, cyclophilin b, colorectal associated antigen (CRC)--C017-1A/GA733, carcinoembryonic antigen (CEA), CAP-1, CAP-2, etv6, AML1, prostate specific antigen (PSA), PSA-1, PSA-2, PSA-3, prostate-specific membrane antigen (PSMA), T cell receptor/CD3-zeta chain and CD20. The cancer antigen may be selected from the group consisting of MAGE-A1, MAGE-A2, MAGE-A3, MAGE-A4, MAGE-A5, MAGE-A6, MAGE-A7, MAGE-A8, MAGE-A9, MAGE-A 10, MAGE-A 11, MAGE-A 12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4 and MAGE-C5. The cancer antigen may be selected from the group consisting of GAGE-1, GAGE-2, GAGE-3, GAGE-4, GAGE-5, GAGE-6, GAGE-7, GAGE-8 and GAGE-9. The cancer antigen may be selected from the group consisting of BAGE, RAGE, LAGE-1, NAG, GnT-V, MUM-1, CDK4, tyrosinase, p53, MUC family, HER2/neu, p21ras, RCAS1, α-fetoprotein, E-cadherin, α-catenin, β-catenin, γ-catenin, p120ctn, gp100Pmel117, PRAME, NY-ESO-1, cdc27, adenomatous polyposis coli protein (APC), fodrin, Connexin 37, Ig-idiotype, p15, gp75, GM2 ganglioside, GD2 ganglioside, human papilloma virus proteins, Smad family of tumor antigens, 1mp-1, P1A, EBV-encoded nuclear antigen (EBNA)-1, brain glycogen phosphorylase, SSX-1, SSX-2 (HOM-MEL-40), SSX-3, SSX-4, SSX-5, SCP-1 and CT-7, CD20 and c-erbB-2. Other cancer antigens may be operably linked to a synthetic promoter, as provided herein.

In some embodiments, a protein or polypeptide-based therapeutic molecule is a fusion protein. A fusion protein is a protein comprising two heterologous proteins, protein domains, or protein fragments, that are covalently bound to each other, either directly or indirectly *(e.g.,* via a linker), via a peptide bond. In some embodiments, a fusion protein is encoded by a nucleic acid comprising the coding region of a protein in frame with a coding region of an additional protein, without intervening stop codon, thus resulting in the translation of a single protein in which the proteins are fused together.

### EXAMPLES

### Example 1: Sequestron design and validation.

Engineered mammalian systems hold great promise for developmental biology, drug development, disease models, and transplantation. One possibility is to harness and control the intricate dynamics of cellular behavior, particularly the multitude of components whose levels continuously evolve in time and can be difficult to detect. The general purpose of this technology is to enable sense and control of cellular behavior by regulating gene expression via endoribonucleases in response to spatial-temporal changes in endogenous miRNA levels. These responses could include incorporation of permanently switching gene expression on or off, transiently modulating gene expression in the shape of pulses and dips in expression, and more complex oscillatory or higher-order harmonics. These regulated gene expression events may encode both RNA and/or proteins to be used in cascading regulation, partitioning to be localized within the cell or secreted extracellularly.

This regulated gene expression platform provides miRNA-sensing circuits with constitutive ERNs to regulate gene expression. Specifically, circuits that monitor cell state by detecting and responding to expression of endogenous miRNAs were developed. miRNA-sensing circuits comprise miRNA target sequences added to the 3'-or 5'-untranslated regions (UTRs) of output genes or repressors of output genes. Integrated with this miRNA sensing program, a multi miRNA inputs-based gene regulating motif for miRNAs using a "endoRNase (CasE) repression" module was developed. This module allows expression of output molecule(s) only if the desired miRNA(s) are present at high levels, but efficiently represses expression of the output molecule if the desired miRNA levels are low **(****FIGs. 1A-****1B).** This system was validated in mammalian cell lines to sense ectopic miRNA-122 and then express an output fluorescent marker, mKate2 **(****Fig. 1C****).**

More generally, this circuit technology produced a class of synthetic circuits termed "sequestrons" tailored to a) process multi-input gene patterns that evolve over time, and b) control multiple outputs, in cells. Sequestrons can have both graded and weighted input and output responses, allowing for unprecedented capacity to match and process complex inputs in a compact, scalable architecture. The sequestron platform leverages both prior miRNA-based cell state classifiers (WO 2019/027414A1) and PERSIST platform (DiAndreth et al. bioRxiv. 2019. doi: 10.1101/2019.12.15.867150) architectures to yield unparalleled multi-input/multi-output functionality.

Sequestrons are based on a combination of endoribonucleases, RNA degradation domains, miRNA sensing operators, and payloads of interest, which are arranged in two primary components: a sensor circuit and a signal circuit. The sensor circuit encodes a) a repressor molecule, such as an endoribonuclease, and b) one or more target sequences for a first set of miRNAs. The signal circuit encodes a) a repressor recognition sequence, such as an RNA sequence that can be cleaved by the repressor/endoribonuclease encoded by the sensor circuit, and b) an output molecule. Both circuits are controlled by constitutive promoters, such that mRNA is produced by both, with regulation of output molecule expression occurring at the level of translation. In the absence of miRNA, the sensor circuit produces the endoribonuclease, which efficiently cleaves the mRNA produced by the signal circuit, thereby preventing translation of the output molecule. If any of the first set of miRNAs are present, however, the mRNA encoding the endoribonuclease is degraded by the target-directed mRNA degradation (TDMD) pathway, preventing translation of the endoribonuclease and subsequent cleavage of the mRNA encoding the output molecule. This arrangement of regulatory components in a sequestron ensures that the output molecule is expressed only if at least one of the first set of miRNAs is present in a cell. Thus, this sequestron is equivalent to an OR gate, with the first set of miRNAs as inputs and expression of the output molecule as an output.

Sequestrons can easily scale to be N-input, M-output circuits by including additional payloads of interest or miRNA operator sites as sensors. Additionally, conditional logic can be applied on inputs and outputs to yield sophisticated expression programs as well as include feedback. An example sequestron with one input (miR-122) and four outputs (Prox1, ATF5, Cyp3A4, and mKO2) is shown in **FIGs. 2A****.** Both elements of the sequestron are integrated in cells of a liver organoid. BFP is expressed constitutively as a control reporter protein, while mKO2 fluorescence is observed only in cells containing high levels of miR-122 **(****FIG. 2B****).** During the course of development, qRT-PCR measurement of mKO2 is correlated with expression of liver maturation genes that are also activated by the presence of miR-122, *Prox1, ATF5,* and *Cyp3A4* **(****FIG. 2C****).**

Sequestrons are particularly useful in situations where input conditions are logistically challenging, such as targeted therapeutics that are cell-type dependent, in differentiation pathways for generation or organoids, or where biomanufacturers require finely tuned control of gene expression or output molecule production. In all of these contexts, the ability to create semi- or fully autonomous sequestrons that react to the inputs of its conditions, rather than external cues, would enable control on a cellular level that is currently not possible. Additionally, the use of endoribonuclease activity and miRNA sensing at the RNA level supports implementations delivered as an RNA therapeutic, opening up new possibilities for treatment of multiple diseases or disorders.

### Example 2: Implementation of sequestrons in liver organoids for differentiation of mesoderm into T cells.

To guide the mesoderm lineage cells towards various immune cells, multi-step developmental programs are used that leverage miRNA-sensing technology to detect cell states and trigger overexpression of specific transcriptional factors. First, vascularized Gata6-hiPSC liver organoids were generated, which contain mesoderm and hematopoietic progenitor cells [1]. Expression of master regulator GATA6 was induced, resulting in a complex multi-cellular, multi-germ layer organoid comprising liver-associated cell types co-developing with hematopoietic and stromal cells [2]. The remarkable range of cell types generated included cell types from each germ layer. Previous approaches [3-4] relied on treatment with external signaling factors, a method that inherently introduces variability across differentiation stages and is usually limited to the derivation of a select few cell types within the hepatic lineage [3-4]. By contrast, this system mimics the natural environment in that required non-hepatic cells are generated, including hepatocytes, cholangiocytes, endothelial, hematopoietic, stellate, and pericyte-like cells, overcoming deficient vascularization and other limitations of 3D systems. Organoids were grown and observed for signs of maturation **(****FIGs. 3A-3B****).** Organoids grew to be multiple centimeters in size **(****FIGs. 3C-3D****)** and generated vascularized networks comprising CD34⁺ cells **(****FIG. 3E****).**

**Integrated miRNA sensors.** An infrastructure for rapid construction of large (over 25kb) mammalian genetic circuits and integration into chromosomal "landing pads" in various cell lines was developed [5-7]. Circuits that monitor cell state by detecting and responding to expression of endogenous miRNAs, including classifier circuits that distinguish between cancer cells (HeLa) and healthy cells and induce apoptosis only in cancer cells, were also developed **(****FIG. 4G****)** [5,8]. Classifier circuits comprise miRNA target sites added to the 3'- or 5'-untranslated regions (UTRs) of output genes or repressors of output genes. To produce an output protein only when levels of a particular miRNA are low, target sites for that miRNA were appended to the UTR of the output gene. Such a "low sensor" suppresses production of the output protein via RNA interference if the miRNA level is high. A "high sensor" motif for miRNAs using a "endoRNase (CasE) repression" module was also constructed. This "high sensor" allows output expression only if miR-122 is present at high levels, but efficiently represses expression of the output molecule if the miR-122 level is low **(****FIGs. 4A-B).** Stable integration into hiPSC landing pads was validated, as were long-term expression of the output molecules of the high miRNA-122 sensor that detects hepatocyte-like cells during organoid differentiation **(****FIGs. 4C-F).** Also shown is a liver organoid at day 14 of maturation, in which this sensor expressing mKO2 when miR-122 is high. By using high/low sensors to control outputs with desired effector functions, classifier circuits guide programed responses to multi-input miRNA profiles with more precision than approaches using cell-type specific promoters [9-12]. To help create such programs, a library of 620 miRNA sensors was developed, which was used to demonstrate differential miRNA activity in different cell types **(****Fig. 4H****)** [13].

Here, sensors integrated into liver organoids incorporate the sensing of miRNAs specific to mesoderm, which gives rise to blood vessels and lymphatic tissue [14].

**Guided differentiation from mesoderm to hemangioblasts.** GATA6-iPSCs mesoderm is sensed using a miR-483-3p high sensor, and differentiation of hemangioblasts is conducted via Ets variant 2 (ETV2) **(****FIG. 5A****).** miR-483-3p shows exclusive expression in mesoderm [15]. This miRNA sensor design is based on the module described above with endoRNase (CasE) and CasE cleavage site in the 3'-UTR. miR-483-3p high sensor regulates expression of transcription factor ETV2 that guides mesoderm to hemangioblast differentiation **(****FIG. 5C****).** ETV2 is essential for hemangioblasts development and sufficient to induce endothelial gene expression in stem cells [16]. KDR, PDGFRA, MEOX1, CD34, Flk, Brachyury, and VE-cadherin are used as biomarkers for mesoderm and hemangioblasts.

**Guided differentiation from hemangioblasts to hematopoietic progenitor.** A sensor for high levels of miR-142-3p **(****FIG. 5D****),** which is abundant specifically in hematopoietic cells, is used to detect completion of differentiation to hemangioblasts [17]. This sensor ectopically overexpresses Runt-related transcription factor 1 (RUNX1), guiding individual cells to the hematopoietic lineage. RUNX1 is pivotal for endothelial to hematopoietic transition (EHT) [18], definitive hematopoietic development, and T cell development. Ter119, CD31, c-Kit, and CD45 are used to assay hematopoietic stem and progenitor cells.

**Guided differentiation from hematopoietic progenitor to phenotypic pre-thymic progenitors and immune cells.** Emerging hematopoietic progenitors with T lineage potential are guided by co-expressing additional endogenous transcriptional factor HOXA9 [18] as the output of miR-142-3p high sensor, completing the multi-step differentiation to T cells **(****FIGs. 5A** and **5D****).** Non-T hematopoietic lineages are suppressed by engineered expression of hDLL4 Notch ligands [53 19], and the correct phenotype of differentiated cells is validated by staining with Lin, c-kit, CD127, CD135, CD3, CD4 and CD8.

Gene sensor circuits that co-express miRNAs and/or guide RNA may be integrated downstream from endogenous genes, to prevent silencing during extended periods of organoid growth. Other major hematopoietic transcription factors, such as Scl, Lyl1, Lmo2, Gata2, Meis1, Erg, Gfilb Hoxa5, Hoxa7, Hoxa10, Ikzf1, and Setbp1, may be used to guide differentiation of mesoderm cells into T cells.

### References

[1] Kubes, P. and Jenne, C., 2018. Immune responses in the liver. Annual Review of Immunology, 36, pp.247-277.
[2] Guye, P., Ebrahimkhani, M.R., Kipniss, N., Velazquez, J.J., Schoenfeld, E., Kiani, S., Griffith, L.G. and Weiss, R., 2016. Genetically engineering self-organization of human pluripotent stem cells into a liver bud-like tissue using Gata6. Nature Communications, 7(1), pp.1-12.
[3] Sartipy P, Björquist P (2011) Concise review: Human pluripotent stem cell-based models for cardiac and hepatic toxicity assessment. Stem Cells, 29(5):744-748.
[4] Funakoshi, N., Duret, C., Pascussi, J.M., Blanc, P., Maurel, P., Daujat-Chavanieu, M. and Gerbal-Chaloin, S., 2011. Comparison of hepatic-like cell production from human embryonic stem cells and adult liver progenitor cells: CAR transduction activates a battery of detoxification genes. Stem Cell Reviews and Reports, 7(3), pp.518-531.
[5] Wroblewska, L., Kitada, T., Endo, K., Siciliano, V., Stillo, B., Saito, H. and Weiss, R., 2015. Mammalian synthetic circuits with RNA binding proteins for RNA-only delivery. Nature Biotechnology, 33(8), pp.839-841.
[6] Guye, P., Li, Y., Wroblewska, L., Duportet, X. and Weiss, R., 2013. Rapid, modular and reliable construction of complex mammalian gene circuits. Nucleic acids research, p.gkt605.
[7] Duportet X. 2014. Developing new tools and platforms for mammalian synthetic biology: from the assembly and chromosomal integration of complex DNA circuits to the engineering of artificial intercellular communication systems. Dissertation. Université Paris Diderot (Paris 7).
[8] Xie Z, Wroblewska L, Prochazka L, Weiss R, Benenson Y (2011) Multi-input RNAi-based logic circuit for identification of specific cancer cells. Science (80- ) 333(6047):1307-1311.
[9] Pei, Y., Sierra, G., Sivapatham, R., Swistowski, A., Rao, M.S. and Zeng, X., 2015. A platform for rapid generation of single and multiplexed reporters in human iPSC lines. Scientific Reports, 5(1), pp.1-10.
[10] He, X., Tan, C., Wang, F., Wang, Y., Zhou, R., Cui, D., You, W., Zhao, H., Ren, J. and Feng, B., 2016. Knock-in of large reporter genes in human cells via CRISPR/Cas9-induced homology-dependent and independent DNA repair. Nucleic Acids Research, 44(9), pp.e85-e85.
[11] Oceguera-Yanez, F., Kim, S.I., Matsumoto, T., Tan, G.W., Xiang, L., Hatani, T., Kondo, T., Ikeya, M., Yoshida, Y., Inoue, H. and Woltjen, K., 2016. Engineering the AAVS1 locus for consistent and scalable transgene expression in human iPSCs and their differentiated derivatives. Methods, 101, pp.43-55.
[12] Merkle, F.T., Neuhausser, W.M., Santos, D., Valen, E., Gagnon, J.A., Maas, K., Sandoe, J., Schier, A.F. and Eggan, K., 2015. Efficient CRISPR-Cas9-mediated generation of knockin human pluripotent stem cells lacking undesired mutations at the targeted locus. Cell Reports, 11(6), pp.875-883.
[13] Gam, J.J., Babb, J. and Weiss, R., 2018. A mixed antagonistic/synergistic miRNA repression model enables accurate predictions of multi-input miRNA sensor activity. Nature Communications, 9(1), pp.1-12.
[14] Anastassova-Kristeva, M., 2003. The origin and development of the immune system with a view to stem cell therapy. Journal of Hematotherapy & Stem Cell Research, 12(2), pp.137-154.
[15] Ishikawa, D., Diekmann, U., Fiedler, J., Just, A., Thum, T., Lenzen, S. and Naujok, O., 2017. miRNome profiling of purified endoderm and mesoderm differentiated from hESCs reveals functions of miR-483-3p and miR-1263 for cell-fate decisions. Stem Cell Reports, 9(5), pp.1588-1603.
[16] van Bueren, K.L. and Black, B.L., 2012. Regulation of endothelial and hematopoietic development by the ETS transcription factor Etv2. Current Opinion in Hematology, 19(3), pp.199-205.
[17] Nimmo, R., Ciau-Uitz, A., Ruiz-Herguido, C., Soneji, S., Bigas, A., Patient, R. and Enver, T., 2013. MiR-142-3p controls the specification of definitive hemangioblasts during ontogeny. Developmental Cell, 26(3), pp.237-249.
[18] Guo, R., Hu, F., Weng, Q., Lv, C., Wu, H., Liu, L., Li, Z., Zeng, Y., Bai, Z., Zhang, M. and Liu, Y., 2020. Guiding T lymphopoiesis from pluripotent stem cells by defined transcription factors. Cell Research, 30(1), pp.21-33.
[19] Mohtashami, M., Shah, D.K., Nakase, H., Kianizad, K., Petrie, H.T. and Zúñiga-Pflücker, J.C., 2010. Direct comparison of Dl11-and Dll4-mediated Notch activation levels shows differential lymphomyeloid lineage commitment outcomes. The Journal of Immunology, 185(2), pp.867-876.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the scope of the claims.

### EQUIVALENTS

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.
All references, patents and patent applications disclosed herein are incorporated by reference with respect to the subject matter for which each is cited, which in some cases may encompass the entirety of the document.

The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, *i.e.,* elements that are conjunctively present in some cases and disjunctively present in other cases. Multiple elements listed with "and/or" should be construed in the same fashion, *i.e.,* "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, *i.e.,* the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (*i.e.* "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

It discloses the following items:
1. A sequestron comprising:
   (i) a sensor circuit comprising a first constitutive promoter operably linked to a nucleic acid sequence encoding:
      (a) a nucleic acid sequence encoding a repressor; and
      (b) one or more target sequences for a first set of one or more miRNAs; and
   (ii) a signal circuit comprising a second constitutive promoter operably linked to a nucleic acid sequence encoding:
      (a) a repressor recognition sequence that is capable of being bound or cleaved by the repressor of (i)(a); and
      (b) a nucleic acid sequence encoding an output molecule.
2. The sequestron of item 1, wherein the one or more target sequences for the first set of one or more miRNAs of (i)(b) are downstream from the nucleic acid sequence encoding the repressor of (i)(a).
3. The sequestron of item 1 or item 2, wherein the repressor recognition sequence of (ii)(a) is upstream from the nucleic acid sequence encoding the output molecule of (ii)(b).
4. The sequestron of any one of items 1-3, wherein the nucleic acid sequence encoded by the signal circuit of (ii) further comprises one or more target sequences for a second set of one or more miRNAs.
5. The sequestron of item 4, wherein the one or more target sequences for the second set of one or more miRNAs are downstream from the nucleic acid sequence encoding the output molecule.
6. The sequestron of any one of items 1-5, wherein the sequestron comprises a plurality of the signal circuit of (ii).
7. The sequestron of item 6, wherein each of the plurality of signal circuits comprises a target sequence for a different miRNA of the second set of miRNAs, wherein the target sequence is not present on the other signal circuits.
8. The sequestron of any one of items 1-7, wherein the sequestron comprises a plurality of the sensor circuit of (i).
9. The sequestron of item 8, wherein each of the plurality of sensor circuits comprises a target sequence for a different miRNA of the first set of miRNAs, wherein the target sequence is not present on the other sensor circuits.
10. The sequestron of any one of items 1-9, wherein the repressor is an endoribonuclease, an RNAi molecule, or a ribozyme.
11. The sequestron of any one of items 1-10, wherein the repressor is a CRISPR endoribonuclease, and the repressor recognition sequence is a CRISPR endoribonuclease recognition sequence.
12. The sequestron of item 11, wherein the CRISPR endoribonuclease is Cas6, Csy4, CasE, Cse3, LwaCas13a, PspCas13b, RanCas13b, PguCas13b, or RfxCas13d.
13. The sequestron of any one of items 1-8, wherein the first and/or second constitutive promoter is an hEF1-alpha promoter.
14. A composition comprising a plurality of the sequestron of any one of items 1-13, wherein:
   (A) the nucleic acid sequence of (i)(a) of each of the plurality of sequestrons encodes a different repressor;
   (B) the repressor recognition sequence of (ii)(a) of each of the plurality of sequestrons comprises a different nucleic acid sequence;
   (C) the repressor encoded by the sensor circuit of each of the plurality of sequestrons is capable of binding or cleaving the repressor recognition sequence of the signal circuit of the same sequestron; and
   (D) the repressor encoded by the sensor circuit of each sequestron is not capable of binding or cleaving the repressor recognition sequence of a different sequestron.
15. A composition comprising the sequestron of any one of items 1-13 or a plurality of the sequestron of any one of items 1-13.
16. The composition of item 14 or 15, further comprising a pharmaceutically acceptable excipient.
17. A cell comprising the sequestron of any one of items 1-13 or a plurality of the sequestron of any one of items 1-13.
18. The cell of item 17, wherein the cell is a prokaryotic cell.
19. The cell of item 18, wherein the prokaryotic cell is a bacterial cell.
20. The cell of item 17, wherein the cell is a eukaryotic cell.
21. The cell of item 20, wherein the eukaryotic cell is a plant cell, an insect cell, or a mammalian cell.
22. The cell of item 20, wherein the eukaryotic cell is a human cell.
23. The cell of any one of items 17-22, wherein the cell is a diseased cell.
24. The cell of item 23, wherein the cell is a cancer cell.
25. The cell of any one of items 17-24, wherein the cell expresses any one of the first set of microRNAs.
26. A method comprising maintaining the cell of any one of items 17-25 in culture.
27. The method of item 26, further comprising detecting the output molecule.
28. The method of item 27, further comprising classifying the cell.
29. A method comprising delivering the sequestron of any one of items 1-13 or the composition of any one of items 14-16 to a cell.
30. The method of item 29, further comprising detecting the output molecule.
31. The method of item 30, further comprising classifying the cell.
32. A method of treating a disease or disorder, the method comprising delivering the sequestron of any one of items 1-13 or the composition of any one of items 14-16 to a subject in need thereof, wherein the output molecule is a therapeutic molecule that treats the disease or disorder.
33. A method of diagnosing a disease or disorder, the method comprising administering an effective amount of the sequestron of any one of items 1-13 or the composition of any one of items 14-16 to a subject.
34. The method of item 33, further comprising detecting the output molecule.

## Claims

1. An organoid comprising one or more sequestrons, each sequestron comprising:
(i) a sensor circuit comprising a first constitutive promoter operably linked to a nucleic acid sequence encoding:
(a) a nucleic acid sequence encoding a repressor; and
(b) one or more target sequences for a first set of one or more miRNAs; and
(ii) a signal circuit comprising a second constitutive promoter operably linked to a nucleic acid sequence encoding:
(a) a repressor recognition sequence that is capable of being cleaved by the repressor of (i)(a); and
(b) a nucleic acid sequence encoding an output molecule,
wherein the repressor is an endoribonuclease or a ribozyme.

2. A cell comprising one or more sequestrons, each sequestron comprising:
(i) a sensor circuit comprising a first constitutive promoter operably linked to a nucleic acid sequence encoding:
(a) a nucleic acid sequence encoding a repressor; and
(b) one or more target sequences for a first set of one or more miRNAs; and
(ii) a signal circuit comprising a second constitutive promoter operably linked to a nucleic acid sequence encoding:
(a) a repressor recognition sequence that is capable of being bound or cleaved by the repressor of (i)(a); and
(b) a nucleic acid sequence encoding an output molecule,
wherein the repressor is an endoribonuclease or a ribozyme.

3. The organoid of claim 1 or the cell of claim 2,
(A) wherein the one or more target sequences for the first set of one or more miRNAs of (i)(b) are downstream from the nucleic acid sequence encoding the repressor of (i)(a); and/or
(B) wherein the repressor recognition sequence of (ii)(a) is upstream from the nucleic acid sequence encoding the output molecule of (ii)(b); and/or
(C) wherein the nucleic acid sequence encoded by the signal circuit of (ii) further comprises one or more target sequences for a second set of one or more miRNAs, wherein optionally the one or more target sequences for the second set of one or more miRNAs are downstream from the nucleic acid sequence encoding the output molecule; and/or
(D) wherein the sequestron comprises a plurality of the signal circuit of (ii), wherein optionally each of the plurality of signal circuits comprises a target sequence for a different miRNA of the second set of miRNAs, wherein the target sequence is not present on the other signal circuits; and/or
(E) wherein the sequestron comprises a plurality of the sensor circuit of (i), wherein optionally each of the plurality of sensor circuits comprises a target sequence for a different miRNA of the first set of miRNAs, wherein the target sequence is not present on the other sensor circuits.

4. The organoid of claim 1 or claim 3, or the cell of claim 2 or claim 3, wherein the repressor is a CRISPR endoribonuclease, and the repressor recognition sequence is a CRISPR endoribonuclease recognition sequence, wherein optionally the CRISPR endoribonuclease is Cas6, Csy4, CasE, Cse3, LwaCas13a, PspCas13b, RanCasl3b, PguCasl3b, or RfxCas13d, and/or
wherein the first and/or second constitutive promoter is an hEF1-alpha promoter.

5. The organoid of any one of claims 1 or 3-4, or the cell of any one of claims 2-4, comprising a plurality of sequestrons, wherein:
(A) the nucleic acid sequence of (i)(a) of each of the plurality of sequestrons encodes a different repressor;
(B) the repressor recognition sequence of (ii)(a) of each of the plurality of sequestrons comprises a different nucleic acid sequence;
(C) the repressor encoded by the sensor circuit of each of the plurality of sequestrons is capable of binding or cleaving the repressor recognition sequence of the signal circuit of the same sequestron; and
(D) the repressor encoded by the sensor circuit of each sequestron is not capable of binding or cleaving the repressor recognition sequence of a different sequestron.

6. The organoid of any one of claims 1 or 3-5, wherein the organoid is a liver organoid.

7. The cell of any one of claims 2-5,
(i) wherein the cell is a prokaryotic cell, wherein optionally the prokaryotic cell is a bacterial cell, or
(ii) wherein the cell is a eukaryotic cell, wherein optionally the eukaryotic cell is a plant cell, an insect cell, a mammalian cell, or a human cell.

8. The cell of any one of claims 2-5 or 7, wherein said cell is for use in a method of treating a disease or disorder, the method comprising delivering the cell to a subject in need thereof, wherein the output molecule is a therapeutic molecule that treats the disease or disorder.

9. A method comprising maintaining the organoid of any one of claims 1 or 3-6 in culture.

10. A method for guided differentiation of an organoid, the method comprising:
(1) integrating a first sequestron into induced pluripotent stem cells (iPSCs), optionally wherein the sequestron is stably integrated into the iPSCs, wherein the first sequestron comprises:
(i) a sensor circuit comprising a first constitutive promoter operably linked to a nucleic acid sequence encoding:
(a) a nucleic acid sequence encoding a repressor; and
(b) one or more target sequences for a first set of one or more miRNAs;
and
(ii) a signal circuit comprising a second constitutive promoter operably linked to a nucleic acid sequence encoding:
(a) a repressor recognition sequence that is capable of being cleaved by the repressor of (i)(a); and
(b) a nucleic acid sequence encoding an output molecule,
wherein the repressor is an endoribonuclease or a ribozyme; and
(2) culturing the iPSCs under conditions suitable for an organoid, optionally wherein the organoid is a three-dimensional organoid.

11. The method of claim 10, wherein:
(A) the first set of one or more miRNAs of the sensor circuit of the first sequestron comprise an miRNA associated with a germ layer, optionally wherein the germ layer is mesoderm, further optionally wherein the miRNA associated with mesoderm is miR-483-3p; and
(B) the output molecule of the signal circuit of the first sequestron comprises a transcription factor that guides differentiation of the iPSCs into desired precursor cells, optionally wherein the desired precursor cells are hemangioblasts, further optionally wherein the transcription factor is ETV2.

12. The method of claim 11, wherein a second sequestron is integrated into the iPSCs, optionally wherein the second sequestron is stably integrated into the iPSCs, the second sequestron comprising:
(i) a sensor circuit comprising a first constitutive promoter operably linked to a nucleic acid sequence encoding:
(a) a nucleic acid sequence encoding a repressor; and
(b) one or more target sequences for a first set of one or more miRNAs; and
(ii) a signal circuit comprising a second constitutive promoter operably linked to a nucleic acid sequence encoding:
(a) a repressor recognition sequence that is capable of being cleaved by the repressor of (i)(a); and
(b) a nucleic acid sequence encoding an output molecule,
wherein the repressor is an endoribonuclease or a ribozyme; and
wherein:
(A) the first set of one or more miRNAs of the sensor circuit of the second sequestron comprise an miRNA associated with desired progenitor cells, optionally wherein the desired progenitor cells are hematopoietic progenitor cells, further optionally wherein the miRNA associated with hematopoietic progenitor cells is miR-142-3p; and
(B) the output molecule of the signal circuit of the second sequestron comprises a transcription factor that guides differentiation of desired precursor cells into desired progenitor cells, optionally wherein the desired progenitor cells are hematopoietic progenitor cells, further optionally wherein the transcription factor is RUNX1.

13. The method of claim 12, wherein the output molecule of the signal circuit of the second sequestron further comprises a different transcription factor that guides differentiation of the desired precursor cells into cells of a desired lineage, optionally wherein the cells of a desired lineage are T cells, further optionally wherein the different transcription factor is HOXA9.

14. The method of claim 10, wherein the iPSCs are human iPSCs (hiPSCs), optionally wherein the hiPSCs express GATA6.

15. An induced pluripotent stem cell (iPSC) comprising a first and second sequestron, wherein each sequestron comprises:
(i) a sensor circuit comprising a first constitutive promoter operably linked to a nucleic acid sequence encoding:
(a) a nucleic acid sequence encoding a repressor; and
(b) one or more target sequences for a first set of one or more miRNAs; and
(ii) a signal circuit comprising a second constitutive promoter operably linked to a nucleic acid sequence encoding:
(a) a repressor recognition sequence that is capable of being cleaved by the repressor of (i)(a); and
(b) a nucleic acid sequence encoding an output molecule,
wherein the repressor is an endoribonuclease or a ribozyme; and
wherein:
(A) the first set of one or more miRNAs of the sensor circuit of the first sequestron comprise an miRNA associated with a germ layer, optionally wherein the germ layer is mesoderm, further optionally wherein the miRNA associated with mesoderm is miR-483-3p; and the output molecule of the signal circuit of the first sequestron comprises a transcription factor that guides differentiation of the iPSC into desired precursor cells, optionally wherein the desired precursor cells are hemangioblasts, further optionally wherein the transcription factor is ETV2; and
(B) the first set of one or more miRNAs of the sensor circuit of the second sequestron comprise an miRNA associated with desired progenitor cells, optionally wherein the desired progenitor cells are hematopoietic progenitor cells, further optionally wherein the miRNA associated with hematopoietic progenitor cells is miR-142-3p; and the output molecule of the signal circuit of the second sequestron comprises a transcription factor that guides differentiation of desired precursor cells into desired progenitor cells, optionally wherein the desired progenitor cells are hematopoietic progenitor cells, further optionally wherein the transcription factor is RUNX1;
optionally wherein the output molecule of the signal circuit of the second sequestron further comprises a different transcription factor that guides differentiation of the desired precursor cells into cells of a desired lineage, optionally wherein the cells of a desired lineage are T cells, further optionally wherein the different transcription factor is HOXA9.
